# EUROPEAN PATENT APPLICATION

(11) **EP 2 284 281 A1**
(43) Date of publication of application: **16.02.2011**
(21) Application number: 09723676.4
(22) Date of filing: 12.03.2009
(51) Int. Cl.: C12Q 1/68, C12N 15/09

(54) **REAGENT COMPRISING PRIMER FOR DETECTION OF mRNA FOR CYTOKERATIN-7**

(30) Priority: 28.03.2008 JP 2008088188
(71) Applicant: Sysmex Corporation, Kobe-shi, Hyogo 651-0073 (JP)
(72) Inventor: HIYAMA, Kayo, Kobe-shi Hyogo 651-0073 (JP); NAKABAYASHI, Kazuki, Kobe-shi Hyogo 651-0073 (JP); OTOMO, Yasuhiro, Kobe-shi Hyogo 651-0073 (JP); TAKATA, Hideki, Kobe-shi Hyogo 651-0073 (JP)
(74) Representative: Paemen, Liesbet R.J.
(86) International application number: PCT/JP2009/054769
(87) International publication number: WO 2009/119331

(57) **Abstract**

Disclosed is a primer for detecting mRNA for CK7. The primer comprises a first sequence on its 5'-terminal side and a second sequence on its 3'-terminal side. The first sequence has a length of 10 to 30 nucleotides, and can hybridize with a strand complementary to a first region which is a region contained in the sequence depicted in SEQ ID NO:1. The second sequence has a length of 10 to 30 nucleotides, and can hybridize with a second region which is located on the 3'-terminal side from the first region in the sequence depicted in SEQ ID NO:1.

SEQUENCE LISTING

<110>  Sysmex Corporation

<120>  Primer for detecting CK7mRNA

<130>  07-097

<150>  JP 2008-088188
<151>  2008-03-28

<160>  120

<170>  PatentIn version 3.1

<210>  1
<211>  1753
<212>  DNA
<213>  Homo sapiens

<400>  1
cagccccgcc cctacctgtg gaagcccagc cgcccgctcc cgcggataaa aggcgcggag      60

tgtccccgag gtcagcgagt gcgcgctcct cctcgcccgc cgctaggtcc atcccggccc     120

agccaccatg tccatccact tcagctcccc ggtattcacc tcgcgctcag ccgccttctc     180

gggccgcggc gcccaggtgc gcctgagctc cgctcgcccc ggcggccttg gcagcagcag     240

cctctacggc ctcggcgcct cacggccgcg cgtggccgtg cgctctgcct atgggggccc     300

ggtgggcgcc ggcatccgcg aggtcaccat taaccagagc ctgctggccc cgctgcggct     360

ggacgccgac ccctccctcc agcgggtgcg ccaggaggag agcgagcaga tcaagaccct     420

caacaacaag tttgcctcct tcatcgacaa ggtgcggttt ctggagcagc agaacaagct     480

gctggagacc aagtggacgc tgctgcagga gcagaagtcg gccaagagca gccgcctccc     540

agacatcttt gaggcccaga ttgctggcct tcggggtcag cttgaggcac tgcaggtgga     600

tgggggccgc ctggaggcgg agctgcggag catgcaggat gtggtggagg acttcaagaa     660

taagtacgaa gatgaaatta accaccgcac agctgctgag aatgagtttg tggtgctgaa     720

gaaggatgtg gatgctgcct acatgagcaa ggtggagctg gaggccaagg tggatgccct     780

gaatgatgag atcaacttcc tcaggaccct caatgagacg gagttgacag agctgcagtc     840

ccagatctcc gacacatctg tggtgctgtc catggacaac agtcgctccc tggacctgga     900

cggcatcatc gctgaggtca aggcgcagta tgaggagatg gccaaatgca gccgggctga     960

ggctgaagcc tggtaccaga ccaagtttga gaccctccag gcccaggctg ggaagcatgg    1020

ggacgacctc cggaataccc ggaatgagat ttcagagatg aaccgggcca tccagaggct    1080

gcaggctgag atcgacaaca tcaagaacca gcgtgccaag ttggaggccg ccattgccga    1140

ggctgaggag cgtggggagc tggcgctcaa ggatgctcgt gccaagcagg aggagctgga    1200

agccgccctg cagcggggca agcaggatat ggcacggcag ctgcgtgagt accaggaact 1260

catgagcgtg aagctggccc tggacatcga gatcgccacc taccgcaagc tgctggaggg 1320

cgaggagagc cggttggctg gagatggagt gggagccgtg aatatctctg tgatgaattc 1380

cactggtggc agtagcagtg gcggtggcat tgggctgacc ctcgggggaa ccatgggcag 1440

caatgccctg agcttctcca gcagtgcggg tcctgggctc ctgaaggctt attccatccg 1500

gaccgcatcc gccagtcgca ggagtgcccg cgactgagcc gcctcccacc actccactcc 1560

tccagccacc acccacaatc acaagaagat tcccacccct gcctcccatg cctggtccca 1620

agacagtgag acagtctgga aagtgatgtc agaatagctt ccaataaagc agcctcattc 1680

tgaggcctga gtgatccacg tgaaaaaaaa aaaaaaaaaa aaaaaaaaaa aaaaaaaaaa 1740

aaaaaaaaaa aaa 1753

<210> 2
<211> 20
<212> DNA
<213> Artificial

<220>
<223> Primer designed based on CK7mRNA

<400> 2
gacatctttg aggcccagat 20

<210> 3
<211> 19
<212> DNA
<213> Artificial

<220>
<223> Primer designed based on CK7mRNA

<400> 3
tctttgaggc ccagattgc 19

<210> 4
<211> 19
<212> DNA
<213> Artificial

<220>
<223> Primer desgined based on CK7mRNA

<400> 4
cccagacatc tttgaggcc 19

<210> 5
<211> 19
<212> DNA
<213> Artificial

<220>
<223>  Primer designed based on CK7mRNA

<400>  5
agacggagtt gacagagct                                                    19


<210>  6
<211>  18
<212>  DNA
<213>  Artificial

<220>
<223>  Primer designed based on CK7mRNA

<400>  6
cagagctgca gtcccaga                                                     18


<210>  7
<211>  20
<212>  DNA
<213>  Artificial

<220>
<223>  Primer desinged based on CK7mRNA

<400>  7
cttcctcagg accctcaatg                                                   20


<210>  8
<211>  16
<212>  DNA
<213>  Artificial

<220>
<223>  Primer designed based on CK7mRNA

<400>  8
gcggggcaag caggat                                                       16


<210>  9
<211>  20
<212>  DNA
<213>  Artificial

<220>
<223>  Primer designed based on CK7mRNA

<400>  9
cccagatctc cgacacatct                                                   20


<210>  10
<211>  17
<212>  DNA
<213>  Artificial

<220>
<223>  Primer designed based on CK7mRNA

<400>  10
acagagctgc agtccca                                                    17


<210>  11
<211>  20
<212>  DNA
<213>  Artificial

<220>
<223>  Primer designed based on CK7mRNA

<400>  11
tgccctgaat gatgagatca                                                 20


<210>  12
<211>  19
<212>  DNA
<213>  Artificial

<220>
<223>  Primer desgined based on CK7mRNA

<400>  12
gaggagatgg ccaaatgca                                                  19


<210>  13
<211>  16
<212>  DNA
<213>  Artificial

<220>
<223>  Primer designed based on CK7mRNA

<400>  13
gcggggcaag caggat                                                     16


<210>  14
<211>  35
<212>  DNA
<213>  Artificial

<220>
<223>  Primer designed based on CK7mRNA

<400>  14
tgcatgctcc gcagctccgg gtcagcttga ggcac                                35


<210>  15
<211>  39
<212>  DNA
<213>  Artificial

<220>

<223>  Primer designed based on CK7mRNA

<400>  15
gtcctccacc acatcctgca tggggtcagc ttgaggcac                              39


<210>  16
<211>  39
<212>  DNA
<213>  Artificial

<220>
<223>          Primer designed based on CK7mRNA

<400>  16
caggtccagg gagcgactgt tcccagatct ccgacacat                              39


<210>  17
<211>  40
<212>  DNA
<213>  Artificial

<220>
<223>  Primer designed based on CK7mRNA

<400>  17
agcgatgatg ccgtccaggt cgacacatct gtggtgctgt                             40


<210>  18
<211>  41
<212>  DNA
<213>  Artificial

<220>
<223>          Primer designed based on CK7mRNA

<400>  18
ttgtccatgg acagcaccac agagacggag ttgacagagc t                           41


<210>  19
<211>  39
<212>  DNA
<213>  Artificial

<220>
<223>          Primer designed based on CK7mRNA

<400>  19
gcgatctcga tgtccagggc ccggcagctg cgtgagtac                              39


<210>  20
<211>  40
<212>  DNA
<213>  Artificial

<220>
<223>  Primer designed based on CK7mRNA

<400>  20
caggtccagg gagcgactgt agtcccagat ctccgacaca                              40


<210>  21
<211>  42
<212>  DNA
<213>  Artificial

<220>
<223>  Primer designed based on CK7mRNA

<400>  21
ctgcgccttg acctcagcga tggtgctgtc catggacaac ag                           42


<210>  22
<211>  40
<212>  DNA
<213>  Artificial

<220>
<223>  Primer designed based on CK7mRNA

<400>  22
ctgcgccttg acctcagcgg tgctgtccat ggacaacagt                              40


<210>  23
<211>  41
<212>  DNA
<213>  Artificial

<220>
<223>  Primer designed based on CK7mRNA

<400>  23
ctgcgccttg acctcagcga gacacatctg tggtgctgtc c                            41


<210>  24
<211>  42
<212>  DNA
<213>  Artificial

<220>
<223>  Primer designed based on CK7mRNA

<400>  24
cagggagcga ctgttgtcca atgagacgga gttgacagag ct                           42


<210>  25
<211>  40
<212>  DNA
<213>  Artificial

<220>
<223>         Primer designed based on CK7mRNA

<400>  25
cgtccccatg cttcccagcc ctgaagcctg gtaccagacc                              40

<210>  26
<211>  39
<212>  DNA
<213>  Artificial

<220>
<223>  Primer designed based on CK7mRNA

<400>  26
tcgtccccat gcttcccagc tgaagcctgg taccagacc                               39

<210>  27
<211>  38
<212>  DNA
<213>  Artificial

<220>
<223>  Primer designed based on CK7mRNA

<400>  27
cgtccccatg cttcccagct gaagcctggt accagacc                                38

<210>  28
<211>  39
<212>  DNA
<213>  Artificial

<220>
<223>  Primer designed based on CK7mRNA

<400>  28
tcgtccccat gcttcccagc tgaagcctgg taccagacc                               39

<210>  29
<211>  37
<212>  DNA
<213>  Artificial

<220>
<223>  Primer designed based on CK7mRNA

<400>  29
cgatctcgat gtccagggcc acggcagctg cgtgagt                                 37

<210>  30
<211>  38
<212>  DNA
<213>  Artificial

<220>
<223>  Primer designed based on CK7mRNA

<400>  30

cgatctcgat gtccagggcc aggcagctgc gtgagtac                                38

<210>  31
<211>  39
<212>  DNA
<213>  Artificial

<220>
<223>  Primer designed based on CK7mRNA

<400>  31
ggtggcgatc tcgatgtcca gcggcagctg cgtgagtac                              39

<210>  32
<211>  37
<212>  DNA
<213>  Artificial

<220>
<223>  Primer designed based on CK7mRNA

<400>  32
ggtggcgatc tcgatgtcca gcggcagctg cgtgagt                                37

<210>  33
<211>  39
<212>  DNA
<213>  Artificial

<220>
<223>  Primer designed based on CK7mRNA

<400>  33
aaccaccgca cagctgctga ggcagcatcc acatccttc                              39

<210>  34
<211>  37
<212>  DNA
<213>  Artificial

<220>
<223>  Primer designed based on CK7mRNA

<400>  34
aaccaccgca cagctgctgg cagcatccac atccttc                                37

<210>  35
<211>  41
<212>  DNA
<213>  Artificial

<220>
<223>  Primer designed based on CK7mRNA

<400>  35
gcgcagtatg aggagatggc cctggagggt ctcaaacttg g                           41

<210>  36
<211>  42
<212>  DNA
<213>  Artificial

<220>
<223>                 Primer designed based on CK7mRNA

<400>  36
gcgcagtatg aggagatggc cagagggtct caaacttggt ct                          42


<210>  37
<211>  42
<212>  DNA
<213>  Artificial

<220>
<223>         Primer designed based on CK7mRNA

<400>  37
ggtcaaggcg cagtatgagg aggggtctca aacttggtct gg                          42


<210>  38
<211>  40
<212>  DNA
<213>  Artificial

<220>
<223>         Primer designed based on CK7mRNA

<400>  38
cacctaccgc aagctgctgg tcacagagat attcacggct                             40


<210>  39
<211>  44
<212>  DNA
<213>  Artificial

<220>
<223>  Primer designed based on CK7mRNA

<400>  39
gccaaatgca gccgggcttg gagggtctca aacttggtct ggta                        44


<210>  40
<211>  40
<212>  DNA
<213>  Artificial

<220>
<223>  Primer designed based on CK7mRNA

<400>  40
ggacggcatc atcgctgagg gtctcaaact tggtctggta                             40

<210>  41
<211>  43
<212>  DNA
<213>  Artificial

<220>
<223>  Primer designed based on CK7mRNA

<400>  41
ttcagagatg aaccgggcca tccacgctgg ttcttgatgt tgt                          43


<210>  42
<211>  39
<212>  DNA
<213>  Artificial

<220>
<223>  Primer designed based on CK7mRNA

<400>  42
ttcagagatg aaccgggcca tcggcacgct ggttcttga                               39


<210>  43
<211>  40
<212>  DNA
<213>  Artificial

<220>
<223>  Primer designed based on CK7mRNA

<400>  43
ttcagagatg aaccgggcca tcggcacgct ggttcttgat                              40


<210>  44
<211>  45
<212>  DNA
<213>  Artificial

<220>
<223>         Primer designed based on CK7mRNA

<400>  44
cacctaccgc aagctgctgg atcatcacag agatattcac ggctc                        45


<210>  45
<211>  45
<212>  DNA
<213>  Artificial

<220>
<223>  Primer designed based on CK7mRNA

<400>  45
ccgcaagctg ctggagggaa ttcatcacag agatattcac ggctc                        45

<210>  46
<211>  19
<212>  DNA
<213>  Artificial

<220>
<223>  Primer designed based on CK7mRNA

<400>  46
cagctccacc ttgctcatg                                                    19


<210>  47
<211>  19
<212>  DNA
<213>  Artificial

<220>
<223>         Primer designed based on CK7mRNA

<400>  47
agctccacct tgctcatgt                                                    19


<210>  48
<211>  18
<212>  DNA
<213>  Artificial

<220>
<223>  Primer designed based on CK7mRNA

<400>  48
aggtcgtccc catgcttc                                                     18


<210>  49
<211>  17
<212>  DNA
<213>  Artificial

<220>
<223>  Primer designed based on CK7mRNA

<400>  49
cgtccccatg cttccca                                                      17


<210>  50
<211>  17
<212>  DNA
<213>  Artificial

<220>
<223>  Primer designed based on CK7mRNA

<400>  50
tccccatgct tcccagc                                                      17


<210>  51

<211>  17
<212>  DNA
<213>  Artificial

<220>
<223>  Primer designed based on CK7mRNA

<400>  51
caccgccact gctactg                                                    17


<210>  52
<211>  17
<212>  DNA
<213>  Artificial

<220>
<223>         Primer designed based on CK7mRNA

<400>  52
cgtccccatg cttccca                                                    17


<210>  53
<211>  18
<212>  DNA
<213>  Artificial

<220>
<223>  Primer designed based on CK7mRNA

<400>  53
ggtcgtcccc atgcttcc                                                   18


<210>  54
<211>  17
<212>  DNA
<213>  Artificial

<220>
<223>         Primer designed based on CK7mRNA

<400>  54
ggtcgtcccc atgcttc                                                    17


<210>  55
<211>  16
<212>  DNA
<213>  Artificial

<220>
<223>         Primer designed based on CK7mRNA

<400>  55
ggcggcctcc aacttg                                                     16


<210>  56
<211>  16

<212>  DNA
<213>  artificial

<220>
<223>  Primer designed based on CK7mRNA

<400>  56
tggcggcctc caactt                                                    16


<210>  57
<211>  18
<212>  DNA
<213>  Artificial

<220>
<223>        Primer designed based on CK7mRNA

<400>  57
aatggcggcc tccaactt                                                  18


<210>  58
<211>  18
<212>  DNA
<213>  Artificial

<220>
<223>        Primer designed based on CK7mRNA

<400>  58
ccactgctac tgccacca                                                  18


<210>  59
<211>  17
<212>  DNA
<213>  Artificial

<220>
<223>  Primer designed based on CK7mRNA

<400>  59
atggcggcct ccaactt                                                   17


<210>  60
<211>  18
<212>  DNA
<213>  Artificial

<220>
<223>  Primer designed based on CK7mRNA

<400>  60
cactgctact gccaccag                                                  18


<210>  61
<211>  19
<212>  DNA

<213>  Artificial

<220>
<223>         Primer designed based on CK7mRNA

<400>  61
gggcctggag ggtctcaaa                                                    19


<210>  62
<211>  19
<212>  DNA
<213>  Artificial

<220>
<223>         Primer designed based on CK7mRNA

<400>  62
tgggcctgga gggtctcaa                                                    19


<210>  63
<211>  21
<212>  DNA
<213>  Artificial

<220>
<223>  Primer designed based on CK7mRNA

<400>  63
gggcctggag ggtctcaaac t                                                 21


<210>  64
<211>  20
<212>  DNA
<213>  Artificial

<220>
<223>  Primer designed based on CK7mRNA

<400>  64
gggcctggag ggtctcaaac                                                   20


<210>  65
<211>  20
<212>  DNA
<213>  Artificial

<220>
<223>  Primer designed based on CK7mRNA

<400>  65
ggcctggagg gtctcaaact                                                   20


<210>  66
<211>  21
<212>  DNA
<213>  Artificial

<220>
<223>  Primer designed based on CK7mRNA

<400>  66
ggcctggagg gtctcaaact t                                                21


<210>  67
<211>  20
<212>  DNA
<213>  Artificial

<220>
<223>  Primer designed based on CK7mRNA

<400>  67
ctgggcctgg agggtctcaa                                                  20


<210>  68
<211>  20
<212>  DNA
<213>  Artificial

<220>
<223>         Primer designed based on CK7mRNA

<400>  68
tgggcctgga gggtctcaaa                                                  20


<210>  69
<211>  19
<212>  DNA
<213>  Artificial

<220>
<223>  Primer designed based on CK7mRNA

<400>  69
gcctggaggg tctcaaact                                                   19


<210>  70
<211>  19
<212>  DNA
<213>  Artificial

<220>
<223>  Primer designed based on CK7mRNA

<400>  70
tgggcctgga gggtctcaa                                                   19


<210>  71
<211>  20
<212>  DNA
<213>  Artificial

<220>
<223>          Primer designed based on CK7mRNA

<400>  71
gcctggaggg tctcaaactt                                                    20


<210>  72
<211>  21
<212>  DNA
<213>  Artificial

<220>
<223>  Primer designed based on CK7mRNA

<400>  72
tcacgctcat gagttcctgg t                                                  21


<210>  73
<211>  22
<212>  DNA
<213>  Artificial

<220>
<223>  Primer designed based on CK7mRNA

<400>  73
gcttcacgct catgagttcc tg                                                 22


<210>  74
<211>  24
<212>  DNA
<213>  Artificial

<220>
<223>  Primer designed based on CK7mRNA

<400>  74
gcttcacgct catgagttcc tggt                                               24


<210>  75
<211>  22
<212>  DNA
<213>  Artificial

<220>
<223>  Primer designed based on CK7mRNA

<400>  75
cttcacgctc atgagttcct gg                                                 22


<210>  76
<211>  20
<212>  DNA
<213>  Artificial

<220>

<223>  Primer designed based on CK7mRNA

<400>  76
gccagcttca cgctcatgag                                                    20


<210>  77
<211>  23
<212>  DNA
<213>  Artificial

<220>
<223>         Primer designed based on CK7mRNA

<400>  77
gccagcttca cgctcatgag ttc                                                23


<210>  78
<211>  23
<212>  DNA
<213>  Artificial

<220>
<223>         Primer designed based on CK7mRNA

<400>  78
cttcacgctc atgagttcct ggt                                                23


<210>  79
<211>  20
<212>  DNA
<213>  Artificial

<220>
<223>         Primer designed based on CK7mRNA

<400>  79
ggccagcttc acgctcatga                                                    20


<210>  80
<211>  22
<212>  DNA
<213>  Artificial

<220>
<223>                 Primer designed based on CK7mRNA

<400>  80
ccagcttcac gctcatgagt tc                                                 22


<210>  81
<211>  22
<212>  DNA
<213>  Artificial

<220>
<223>         Primer designed based on CK7mRNA

<400>  81
gccagcttca cgctcatgag tt                                                22


<210>  82
<211>  21
<212>  DNA
<213>  Artificial

<220>
<223>  Primer designed based on CK7mRNA

<400>  82
gccagcttca cgctcatgag t                                                 21


<210>  83
<211>  23
<212>  DNA
<213>  Artificial

<220>
<223>  Primer designed based on CK7mRNA

<400>  83
gcttcacgct catgagttcc tgg                                               23


<210>  84
<211>  21
<212>  DNA
<213>  Artificial

<220>
<223>  Primer designed based on CK7mRNA

<400>  84
ggccagcttc acgctcatga g                                                 21


<210>  85
<211>  19
<212>  DNA
<213>  Artificial

<220>
<223>          Primer designed based on CK7mRNA

<400>  85
aggctgcagg ctgagatcg                                                    19


<210>  86
<211>  20
<212>  DNA
<213>  Artificial

<220>
<223>          Primer designed based on CK7mRNA

<400>  86
gaggctgcag gctgagatcg                                                    20


<210>  87
<211>  20
<212>  DNA
<213>  Artificial

<220>
<223>  Primer designed based on CK7mRNA

<400>  87
agaggctgca ggctgagatc                                                    20


<210>  88
<211>  20
<212>  DNA
<213>  Artificial

<220>
<223>  Primer designed based on CK7mRNA

<400>  88
gaggctgcag gctgagatcg                                                    20


<210>  89
<211>  17
<212>  DNA
<213>  Artificial

<220>
<223>         Primer designed based on CK7mRNA

<400>  89
cagaggctgc aggctga                                                       17


<210>  90
<211>  19
<212>  DNA
<213>  Artificial

<220>
<223>  Primer designed based on CK7mRNA

<400>  90
cagaggctgc aggctgaga                                                     19


<210>  91
<211>  21
<212>  DNA
<213>  Artificial

<220>
<223>  Primer designed based on CK7mRNA

<400>  91

cagaggctgc aggctgagat c                                                21

<210>  92
<211>  22
<212>  DNA
<213>  Artificial

<220>
<223>  Primer designed based on CK7mRNA

<400>  92
gctgcaggct gagatcgaca ac                                               22

<210>  93
<211>  20
<212>  DNA
<213>  Artificial

<220>
<223>  Primer designed based on CK7mRNA

<400>  93
gcaggctgag atcgacaaca                                                  20

<210>  94
<211>  20
<212>  DNA
<213>  Artificial

<220>
<223>  Primer designed based on CK7mRNA

<400>  94
gctgcaggct gagatcgaca                                                  20

<210>  95
<211>  21
<212>  DNA
<213>  Artificial

<220>
<223>  Primer designed based on CK7mRNA

<400>  95
gctgcaggct gagatcgaca a                                                21

<210>  96
<211>  20
<212>  DNA
<213>  Artificial

<220>
<223>  Primer designed based on CK7mRNA

<400>  96
ggctgcaggc tgagatcgac                                                  20

<210>  97
<211>  22
<212>  DNA
<213>  Artificial

<220>
<223>  Primer designed based on CK7mRNA

<400>  97
ccggttggct ggagatggag tg                                                22


<210>  98
<211>  18
<212>  DNA
<213>  Artificial

<220>
<223>  Primer designed based on CK7mRNA

<400>  98
agccggttgg ctggagat                                                     18


<210>  99
<211>  22
<212>  DNA
<213>  Artificial

<220>
<223>  Primer designed based on CK7mRNA

<400>  99
cggttggctg gagatggagt gg                                                22


<210>  100
<211>  20
<212>  DNA
<213>  Artificial

<220>
<223>  Primer designed based on CK7mRNA

<400>  100
ccggttggct ggagatggag                                                   20


<210>  101
<211>  20
<212>  DNA
<213>  Artificial

<220>
<223>                  Primer designed based on CK7mRNA

<400>  101
cggttggctg gagatggagt                                                   20

<210>  102
<211>  21
<212>  DNA
<213>  Artificial

<220>
<223>         Primer designed based on CK7mRNA

<400>  102
cggttggctg gagatggagt g                                                  21


<210>  103
<211>  21
<212>  DNA
<213>  Artificial

<220>
<223>  Primer designed based on CK7mRNA

<400>  103
ggttggctgg agatggagtg g                                                  21


<210>  104
<211>  19
<212>  DNA
<213>  Artificial

<220>
<223>         Primer designed based on CK7mRNA

<400>  104
ccggttggct ggagatgga                                                     19


<210>  105
<211>  16
<212>  DNA
<213>  Artificial

<220>
<223>  Primer designed based on CK7mRNA

<400>  105
ttgccgaggc tgagga                                                        16


<210>  106
<211>  39
<212>  DNA
<213>  Artificial

<220>
<223>  Primer designed based on CK7mRNA

<400>  106
cgtccccatg cttcccagcc tgaagcctgg taccagacc                               39

<210>  107
<211>  38
<212>  DNA
<213>  Artificial

<220>
<223>  Primer designed based on CK7mRNA

<400>  107
cgtccccatg cttcccagct gaagcctggt accagacc                                38


<210>  108
<211>  40
<212>  DNA
<213>  Artificial

<220>
<223>         Primer designed based on CK7mRNA

<400>  108
ctgccgtgcc atatcctgct tggctcaagg atgctcgtgc                              40


<210>  109
<211>  42
<212>  DNA
<213>  Artificial

<220>
<223>  Primer designed based on CK7mRNA

<400>  109
cgcagtatga ggagatggcc aactggaggg tctcaaactt gg                           42


<210>  110
<211>  40
<212>  DNA
<213>  Artificial

<220>
<223>  Primer designed based on CK7mRNA

<400>  110
atgaaccggg ccatccagag gcttggcacg ctggttcttg                              40


<210>  111
<211>  42
<212>  DNA
<213>  Artificial

<220>
<223>  Primer designed based on CK7mRNA

<400>  111
ttcagagatg aaccgggcca tcacttggca cgctggttct tg                           42


<210>  112

<211>  42
<212>  DNA
<213>  Artificial

<220>
<223>  Primer designed based on CK7mRNA

<400>  112
tcagagatga accgggccat ccacttggca cgctggttct tg                          42


<210>  113
<211>  43
<212>  DNA
<213>  Artificial

<220>
<223>         Primer designed based on CK7mRNA

<400>  113
ttcagagatg aaccgggcca tccacgctgg ttcttgatgt tgt                         43


<210>  114
<211>  43
<212>  DNA
<213>  Artificial

<220>
<223>  Primer designed based on CK7mRNA

<400>  114
tcagagatga accgggccat cccacgctgg ttcttgatgt tgt                         43


<210>  115
<211>  35
<212>  DNA
<213>  Artificial

<220>
<223>  Primer designed based on CK7mRNA

<400>  115
gtgaagctgg ccctggacac caaccggctc tcctc                                  35


<210>  116
<211>  19
<212>  DNA
<213>  Artificial

<220>
<223>  Primer designed based on CK7mRNA

<400>  116
gaggtcgtcc ccatgcttc                                                    19


<210>  117
<211>  17

<212>  DNA
<213>  Artificial

<220>
<223>        Primer designed based on CK7mRNA

<400>  117
ctcagcctcg gcaatgg                                                    17


<210>  118
<211>  17
<212>  DNA
<213>  Artificial

<220>
<223>  Primer designed based on CK7mRNA

<400>  118
cctcagcctc ggcaatg                                                    17


<210>  119
<211>  17
<212>  DNA
<213>  Artificial

<220>
<223>  Primer designed based on CK7mRNA

<400>  119
tggcggcctc caacttg                                                    17


<210>  120
<211>  18
<212>  DNA
<213>  Artificial

<220>
<223>  Primer designed based on CK7mRNA

<400>  120
gctcccactc catctcca                                                   18

## Description

### TECHNICAL FIELD

The present invention relates to a primer and a primer set used for the detection of cytokeratin-7 (hereinafter referred to as "CK7") mRNA in a sample, as well as to a reagent comprising the same.

### BACKGROUND ART

CK7 is one of cytokeratins which are intermediate filament component proteins found in epithelial cells, and its usefulness as a molecular marker for detecting lymph node metastasis of lung cancer or esophageal cancer has been recently reported. For example, Non-patent Document 1 (Pulte et al., 2005) discloses that lymph node metastasis of non-small-cell lung cancer can be assessed with high specificity by preparing a measurement sample from lymph node of a biological body, and detecting CK7 mRNA in the measurement sample by nucleic acid amplification technology (RT-PCR).
Non-patent Document 1: Pulte et al., CANCER 2005, vol. 104, No.7, p.1453-61.

### SUMMARY OF THE INVENTION

### Technical Problem

As mentioned above, CK7 mRNA is a useful molecular marker for the detection of cancers. In the conventional art, the detection of CK7 mRNA has been carried out by RT-PCT. Although RT-PCR is a nucleic acid amplification method having high sensitivity such that, for example, faint metastasis of cancer cells to lymph node could be detected, the detection by this method is time-consuming (requiring 2 to 4 hours).

Accordingly, the purpose of the present invention is to provide a primer and primer set which allow the detection of CK7 mRNA in a short time compared to the conventional technique.

### Means for Solving the Problems

The present invention provides a primer for detecting CK7 mRNA. The primer comprises a first sequence and a second sequence in its 5' and 3' regions, respectively. The first sequence is 10 to 30 nucleotides long and is capable of hybridizing to a complementary strand to a first region contained in the sequence SEQ ID NO:1. The second sequence is 10 to 30 nucleotides long and capable of hybridizing to a second region which is located at 3' to the first region.

### Effect of the Invention

According to the present invention, a primer and primer set are provided which allow the detection of CK7 mRNA in a short time compared to the conventional technique.

### BRIEF DESCRIPTION OF THE DRAWING

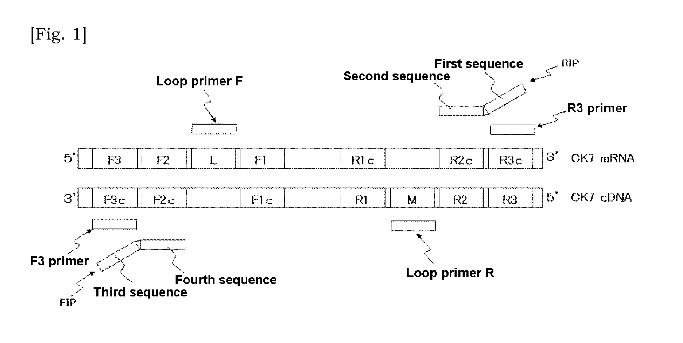

Fig. 1 is a schematic diagram showing the primers for the detection of CK7 mRNA and the nucleic acid regions to which the primers hybridize.

### BEST MODE FOR CARRYING OUT THE INVENTION

As used herein, to "detect" means not only to assess whether or not the target substance of the present invention, CK7 mRNA, is present in a sample, but also to quantify the amount of CK7 mRNA in the sample.
As used herein, "primer" means a polynucleotide having a function to hybridize to a certain region of a nucleic acid to be amplified and to be an origin of amplification reaction by a polymerase (hereinafter referred to as "primer function") in nucleic acid amplification technologies such as LAMP (loop-mediated isothermal amplification) method (see USP 6,410,278 and USP 6,974,670). The nucleic acid to be detected is CK7 mRNA. Thus, it can be detected by nucleic acid amplification reaction comprising reverse transcription reaction (e.g. RT-LAMP).
As used herein, to "hybridize" means whole or a part of a polynucleotide binds via hydrogen bond to whole or a part of another polynucleotide by means of the complementarity of bases in the polynucleotides under stringent conditions.
As used herein, "stringent condition" is a condition usually used by a person skilled in the art upon hybridization reaction of polynucleotides and is not specifically limited as long as it is the condition under which the primer of the present embodiment can hybridize to CK7 mRNA or its cDNA. It is known that the stringency upon hybridization is a function of temperature, salt concentration, the length of primers, the GC content of primers, and the concentration of a chaotropic agent in a hybridization buffer. As the stringent condition, the conditions described in Sambrook, J. et al, 1998, Molecular Cloning: A Laboratory Manual (2nd edition), Cold Spring Harbor Laboratory Press, New York may be used. More specifically, the condition "in a solution containing 50% formamide, 5 x SSC (150 mM NaCl, 15 mM sodium citrate), 50 mM sodium phosphate, pH 7.6, 5 x Denhardt's solution, 10% dextran sulfate and 20µg/ml nucleic acid; and a hybridization temperature of 42°C" may be exemplified without limitation.

The primer according to this embodiment is suitably used for the detection of CK7 mRNA, particularly using RT-LAMP method. The nucleotide sequence of CK7 mRNA is shown in SEQ ID NO:1. Although uracil is contained in the place of thymine in mRNAs, SEQ ID NO:1 is described with thymine (t) in the place of uracil, for the sake of convenience. This sequence is registered in GenBank database under the accession number of NM_005556.

In RT-LAMP, cDNA is synthesized from mRNA by reverse transcription reaction (RT reaction), followed by amplification of the synthesized cDNA by LAMP reaction. When using RT-LAMP for the detection of CK7 mRNA, a primer used can be a polynucleotide which comprises, in its 5' region, a first sequence capable of hybridizing to a region R1 complementary to a region R1 c in CK7 mRNA and, in its 3' region, a second sequence which capable of hybridizing to a region R2c located at downstream (3') to the region R1 c in CK7 mRNA.

The first sequence may be directly linked to the second sequence or may be linked with it via an intervening sequence. The intervening sequence is preferably a sequence having low homology with CK7 mRNA or its cDNA, which is for example 5'-tttt-3'. The intervening sequence is preferably 1 to 50 nucleotides long and more preferably 1 to 40 nucleotides long.

The above primer can act as a reverse inner primer (RIP) among the primers used in RT-LAMP method. In RT-LAMP method, a primer set is used comprising, in addition to RIP, a forward inner primer (FIP) and F3 primer. These FIP and F3 primer are now described further referring to Fig. 1.

Fig. 1 is a schematic diagram showing primers and nucleic acid regions to which the respective primers hybridize. In Fig. 1, the regions F1, F2, L, F1, R1 c, R2c and R3c are comprised in CK7 mRNA, and the regions F3c, F2c, F1 c, R1, M, R2 and R3 are comprised in CK7 cDNA which is a complementary strand to CK7 mRNA. F1, F2, F3, R1, R2 and R3 are complementary to F1c, F2c, F3c, R1 c, R2c and R3c, respectively. These regions are selected in consideration of the detection efficiency of CK7 mRNA, reproducibility of the detection and the like.

The FIP is a polynucleotide which has, in its 5' region, a third sequence capable of hybridizing to the region F1 (complementary region to the region F1c) and, in its 3' side, a fourth sequence capable of hybridizing to the region F2c. The third sequence may be directly linked to the fourth sequence or may be linked with it via the intervening sequence described above.
The F3 primer is a polynucleotide capable of hybridizing to the region F3c which is located downstream (3') to the region F2c in CK7 cDNA.

The primer set may further comprise R3 primer, in addition to FIP, RIP and F3 primer. The R3 primer is a polynucleotide capable of hybridizing to the region R3c which is located downstream (3') to the region R2c in CK7 mRNA.

The primer set may further comprise a loop primer. Such loop primer may include a loop primer F which is a polynucleotide capable of hybridizing to a region L which is located between the regions F2 and F1 in CK7 mRNA, and a loop primer R which is a polynucleotide capable of hybridizing to a region M which is located between the regions R1 and R2 in cDNA of CK7. By using one or both of these loop primers, the amplification of cDNA in LAMP reaction can be further promoted.

The length of each of the first, second, third and fourth sequences, F3 primer, R3 primer and loop primers F and R is not specifically limited so long as the length is suitable for the primer function. According to the fact that known polymerases for catalyzing nucleic acid synthesis reaction recognize primers being approximately 5 nucleotides in length, the length of the above primers is preferably 5 to 100 nucleotides. In consideration of the specificity between the nucleotide sequences of primers and regions to which the primers hybridize, the length of the primers is preferably not less than 10 nucleotides. In consideration of the hybridizing temperature of the primers, it is preferably no more than 30 nucleotides. Thus, the length of FIP and RIP is preferably 10 to 200 nucleotides, and more preferably 20 to 60 nucleotides.

It is preferable that among these primers, at least one primer hybridizes to a region comprising an exon junction in CK7 mRNA. CK7 gene is composed of 9 exons, introns intervening between the exons. When mRNA is synthesized from CK7 gene in cells, the introns are removed during splicing and the exons are directly connected. The joints between exons are called exon junctions. For example, exon 1 and exon 2 are separated by an intron in genome. However, the intron is removed during mRNA synthesis, so that 3' end of exon 1 and 5' end of exon 2 are adjacent in mRNA. The sequence SEQ ID NO:1 consists of 9 exons and therefore contains the following 8 exon junctions (exon junctions 1 to 8).
Exon junction 1 between exons 1 and 2: a joint between positions 451 and 452;
Exon junction 2 between exons 2 and 3: a joint between positions 663 and 664;
Exon junction 3 between exons 3 and 4: a joint between positions 724 and 725;
Exon junction 4 between exons 4 and 5: a joint between positions 820 and 821;
Exon junction 5 between exons 5 and 6: a joint between positions 985 and 986;
Exon junction 6 between exons 6 and 7: a joint between positions 1111 and 1112;
Exon junction 7 between exons 7 and 8: a joint between positions 1332 and 1333;
Exon junction 8 between exons 8 and 9: a joint between positions 1367 and 1368.

The primers capable of hybridizing to the regions comprising the exon junctions hybridize to mRNA, while they have little possibility of hybridizing to CK7 gene in genome. By using such primers, it is possible to prevent non-specific amplification of CK7 gene during mRNA detection by RT-LAMP reaction.

According to the mechanism of RT-LAMP reactions (see USP 6,410,278 and USP 6,974,670), the second sequence in RIP first hybridizes to CK7 mRNA in samples. Therefore, it is preferable that, among the above primers, the second sequence in RIP hybridizes to a region comprising an exon junction in CK7 mRNA; i.e. it is preferable that the region R2c comprises an exon junction. Due to this, non-specific amplification of CK7 gene in the early stage of the reaction can be prevented.

In view of the homology with mRNAs of other cytokeratins (e.g. cytokeratins 6, 8 and the like), detection speed, reproducibility of the detection and the like, it is preferable that the primer hybridizes to a region comprising any of exon junctions 3, 5, 6 and 8. The primer which hybridizes to a region comprising any of exon junctions 1, 2, 4 and 7 may be prepared in consideration of the homology with mRNAs of other cytokeratins (e.g. cytokeratins 5, 6, 8 and the like), detection speed, reproducibility of the detection and the like.

The primer of the present embodiment does not need to be completely complementary to a certain region of CK7 mRNA to which the primer hybridizes, i.e. it may have such complementarity that the hybridization to CK7 mRNA is possible (this is described in USP 4,800,159). Thus, it is sufficient that the primer may be a polynucleotide having the primer function, and it may be a polynucleotide to which one or more mutations such as substitution, deletion, insertion or addition have been introduced in the sequence completely complementary to a certain region. It is preferable that no more than four nucleotides have such mutations. The polynucleotide to which the mutation(s) is introduced preferably has not less than 80% homology, more preferably not less than 90% homology and most preferably not less than 95% homology to the polynucleotide without the mutation(s).

After the primer has hybridized to the target nucleic acid, CK7 mRNA, nucleic acid synthesis is carried out by polymerase starting from the 3'-end of the primer. Thus, when the homology between 3'-end part of the primer and CK7 mRNA is low, the nucleic acid synthesis reaction may not proceed easily. Accordingly, preferably three bases, and more preferably five bases at 3'-end of the primer are completely complementary to the region to which the primer hybridizes.

The regions F3, F2, F1, R1c, R2c, R3c, L and M can be chosen in the sequence of CK7 mRNA represented in SEQ ID NO:1. The examples of these regions are as follows.

### (F1)

The region from positions 619 to 636;
the region from positions 631 to 652;
the region from positions 858 to 879;
the region from positions 873 to 892;
the region from positions 878 to 898;
the region from positions 879 to 898;
the region from positions 893 to 913;
the region from positions 909 to 928;
the region from positions 910 to 928;
the region from positions 1006 to 1025;
the region from positions 1007 to 1025;
the region from positions 1007 to 1026;
the region from positions 1219 to 1240;
the region from positions 1275 to 1295;
the region from positions 1276 to 1296; and the region from positions 1280 to 1300.

### (F2)

The region from positions 574 to 590;
the region from positions 813 to 834;
the region from positions 813 to 882;
the region from positions 816 to 834;
the region from positions 837 to 856;
the region from positions 840 to 857;
the region from positions 851 to 869;
the region from positions 851 to 871;
the region from positions 861 to 882;
the region from positions 863 to 883;
the region from positions 963 to 982;
the region from positions 964 to 982;
the region from positions 1165 to 1182;
the region from positions 1235 to 1252; and
the region from positions 1236 to 1252.

### (F3)

The region from positions 538 to 556;
the region from positions 542 to 561;
the region from positions 546 to 564;
the region from positions 775 to 794;
the region from positions 796 to 815;
the region from positions 816 to 834;
the region from positions 827 to 843;
the region from positions 828 to 845;
the region from positions 840 to 859;
the region from positions 932 to 950;
the region from positions 1134 to 1149; and the region from positions 1213 to 1228.

### (R1c)

The region from positions 680 to 697;
the region from positions 680 to 699;
the region from positions 898 to 917;
the region from positions 916 to 937;
the region from positions 923 to 943;
the region from positions 924 to 945;
the region from positions 941 to 958;
the region from positions 1051 to 1072;
the region from positions 1052 to 1073;
the region from positions 1058 to 1078;
the region from positions 1268 to 1286;
the region from positions 1297 to 1316;
the region from positions 1297 to 1317; and
the region from positions 1303 to 1320.

### (R2c)

The region from positions 721 to 739;
the region from positions 974 to 999;
the region from positions 976 to 995;
the region from positions 978 to 993;
the region from positions 978 to 997;
the region from positions 981 to 1000;
the region from positions 1095 to 1115;
the region from positions 1100 to 1117;
the region from positions 1101 to 1117;
the region from positions 1353 to 1376;
the region from positions 1353 to 1379; and
the region from positions 1354 to 1376.

### (R3c)

The region from positions 741 to 759;
the region from positions 742 to 760;
the region from positions 1007 to 1023;
the region from positions 1009 to 1025;
the region from positions 1011 to 1028;
the region from positions 1012 to 1028;
the region from positions 1012 to 1029;
the region from positions 1012 to 1030;
the region from positions 1117 to 1132;
the region from positions 1117 to 1133;
the region from positions 1118 to 1133;
the region from positions 1118 to 1134;
the region from positions 1118 to 1135;
the region from positions 1131 to 1147;
the region from positions 1132 to 1148;
the region from positions 1356 to 1339;
the region from positions 1383 to 1400;
the region from positions 1384 to 1401; and
the region from positions 1390 to 1406.

### (L)

The region from positions 983 to 1002;
the region from positions 983 to 1003;
the region from positions 984 to 1002;
the region from positions 984 to 1003;
the region from positions 984 to 1004;
the region from positions 985 to 1004;
the region from positions 986 to 1004;
the region from positions 986 to 1005;
the region from positions 987 to 1005;
the region from positions 987 to 1006;
the region from positions 1251 to 1271;
the region from positions 1251 to 1273;
the region from positions 1251 to 1274;
the region from positions 1252 to 1273;
the region from positions 1252 to 1274;
the region from positions 1253 to 1274;
the region from positions 1256 to 1277;
the region from positions 1256 to 1278;
the region from positions 1257 to 1278;
the region from positions 1258 to 1278;
the region from positions 1259 to 1278;
the region from positions 1259 to 1279; and
the region from positions 1260 to 1279.

### (M)

The region from positions 1073 to 1089;
the region from positions 1073 to 1091;
the region from positions 1073 to 1093;
the region from positions 1074 to 1093;
the region from positions 1075 to 1094;
the region from positions 1076 to 1093;
the region from positions 1076 to 1094;
the region from positions 1077 to 1096;
the region from positions 1078 to 1097;
the region from positions 1078 to 1098;
the region from positions 1078 to 1099;
the region from positions 1081 to 1100;
the region from positions 1328 to 1345;
the region from positions 1330 to 1348;
the region from positions 1330 to 1349;
the region from positions 1330 to 1351;
the region from positions 1331 to 1350;
the region from positions 1331 to 1351;
the region from positions 1331 to 1352; and
the region from positions 1332 to 1352.

Based on the above regions, the present primer can be designed. The examples of each primer are shown below. In brackets is described the region in CK7 mRNA (SEQ ID NO:1) to which each primer is identical or complementary.

### (F3 primer)

SEQ ID NO:2: 5'-GACATCTTTGAGGCCCAGAT-3' (identical to the region from positions 542 to 561);
SEQ ID NO:3: 5'-TCTTTGAGGCCCAGATTGC-3' (identical to the region from positions 546 to 564);
SEQ ID NO:4: 5'-CCCAGACATCTTTGAGGCC-3' (identical to the region from positions 538 to 556);
SEQ ID NO:5: 5'-AGACGGAGTTGACAGAGCT-3' (identical to the region from positions 816 to 834);
SEQ ID NO:6: 5'-CAGAGCTGCAGTCCCAGA-3' (identical to the region from positions 828 to 845);
SEQ ID NO:7: 5'-CTTCCTCAGGACCCTCAATG-3' (identical to the region from positions 796 to 815);
SEQ ID NO:8: 5'-GCGGGGCAAGCAGGAT-3' (identical to the region from positions 1213 to 1228);
SEQ ID NO:9: 5'-CCCAGATCTCCGACACATCT-3' (identical to the region from positions 840 to 859);
SEQ ID NO:10: 5'-ACAGAGCTGCAGTCCCA-3' (identical to the region from positions 827 to 843);
SEQ ID NO:11: 5'-TGCCCTGAATGATGAGATCA-3' (identical to the region from positions 775 to 794);
SEQ ID NO:12: 5'-GAGGAGATGGCCAAATGCA-3' (identical to the region from positions 932 to 950); and
SEQ ID NO:13: 5'-GCGGGGCAAGCAGGAT-3' (identical to the region from positions 1213 to 1228).

### (FIP)

SEQ ID NO:14:
   5'-TGCATGCTCCGCAGCTCCGGGTCAGCTTGAGGCAC-3' (the complementary sequence to the region from positions 619 to 636 is linked with the sequence identical to the region from positions 574 to 590);
SEQ ID NO:15:
   5'-GTCCTCCACCACATCCTGCATGGGGTCAGCTTGAGGCAC-3' (the complementary sequence to the region from positions 631 to 652 is linked with the sequence identical to the region from positions 574 to 590);
SEQ ID NO:16:
   5'-CAGGTCCAGGGAGCGACTGTTCCCAGATCTCCGACACAT-3' (the complementary sequence to the region from positions 878 to 898 is linked with the sequence identical to the region from positions 840 to 857);
SEQ ID NO:17:
   5'-AGCGATGATGCCGTCCAGGTCGACACATCTGTGGTGCTGT-3' (the complementary sequence to the region from positions 893 to 913 is linked with the sequence identical to the region from positions 851 to 869);
SEQ ID NO:18:
   5'-TTGTCCATGGACAGCACCACAGAGACGGAGTTGACAGAGCT-3' (the complementary sequence to the region from positions 858 to 879 is linked with the sequence identical to the region from positions 816 to 834);
SEQ ID NO:19:
   5'-GCGATCTCGATGTCCAGGGCCCGGCAGCTGCGTGAGTAC-3' (the complementary sequence to the region from positions 1276 to 1296 is linked with the sequence identical to the region from positions 1235 to 1252);
SEQ ID NO:20:
   5'-CAGGTCCAGGGAGCGACTGTAGTCCCAGATCTCCGACACA-3' (the complementary sequence to the region from positions 879 to 898 is linked with the sequence identical to the region from positions 837 to 856);
SEQ ID NO:21:
   5'-CTGCGCCTTGACCTCAGCGATGGTGCTGTCCATGGACAACAG-3' (the complementary sequence to the region from positions 909 to 928 is linked with the sequence identical to the region from positions 861 to 882);
SEQ ID NO:22:
   5'-CTGCGCCTTGACCTCAGCGGTGCTGTCCATGGACAACAGT-3' (the complementary sequence to the region from positions 910 to 928 is linked with the sequence identical to the region from positions 863 to 883);
SEQ ID NO:23:
   5'-CTGCGCCTTGACCTCAGCGAGACACATCTGTGGTGCTGTCC-3' (the complementary sequence to the region from positions 909 to 928 is linked with the sequence identical to the region from positions 851 to 871);
SEQ ID NO:24:
   5'-CAGGGAGCGACTGTTGTCCAATGAGACGGAGTTGACAGAGCT-3' (the complementary sequence to the region from positions 873 to 892 is linked with the sequence identical to the region from positions 813 to 834);
SEQ ID NO:25:
   5'-CGTCCCCATGCTTCCCAGCCCTGAAGCCTGGTACCAGACC-3' (the complementary sequence to the region from positions 1006 to 1025 is linked with the sequence identical to the region from positions 963 to 982);
SEQ ID NO:26:
   5'-TCGTCCCCATGCTTCCCAGCTGAAGCCTGGTACCAGACC-3' (the complementary sequence to the region from positions 1007 to 1026 is linked with the sequence identical to the region from positions 964 to 982);
SEQ ID NO:27:
   5'-CGTCCCCATGCTTCCCAGCTGAAGCCTGGTACCAGACC-3' (the complementary sequence to the region from positions 1007 to 1025 is linked with the sequence identical to the region from positions 963 to 982);
SEQ ID NO:28:
   5'-TCGTCCCCATGCTTCCCAGCTGAAGCCTGGTACCAGACC-3' (the complementary sequence to the region from positions 1007 to 1026 is linked with the sequence identical to the region from positions 962 to 982);
SEQ ID NO:29:
   5'-CGATCTCGATGTCCAGGGCCACGGCAGCTGCGTGAGT-3' (the complementary sequence to the region from positions 1275 to 1295 is linked with the sequence identical to the region from positions 1235 to 1250);
SEQ ID NO:30:
   5'-CGATCTCGATGTCCAGGGCCAGGCAGCTGCGTGAGTAC-3' (the complementary sequence to the region from positions 1275 to 1295 is linked with the sequence identical to the region from positions 1236 to 1252);
SEQ ID NO:31:
   5'-GGTGGCGATCTCGATGTCCAGCGGCAGCTGCGTGAGTAC-3' (the complementary sequence to the region from positions 1280 to 1300 is linked with the sequence identical to the region from positions 1235 to 1252); and
SEQ ID NO:32:
   5'-GGTGGCGATCTCGATGTCCAGCGGCAGCTGCGTGAGT-3' (the complementary sequence to the region from positions 1280 to 1300 is linked with the sequence identical to the region from positions 1235 to 1250).

### (RIP)

SEQ ID NO:33:
   5'-AACCACCGCACAGCTGCTGAGGCAGCATCCACATCCTTC-3' (the sequence identical to the region from positions 680 to 699 is linked with the complementary sequence to the region from positions 721 to 739);
SEQ ID NO:34:
   5'-AACCACCGCACAGCTGCTGGCAGCATCCACATCCTTC-3' (the sequence identical to the region from positions 680 to 697 is linked with the complementary sequence to the region from positions 721 to 739);
SEQ ID NO:35:
   5'-GCGCAGTATGAGGAGATGGCCCTGGAGGGTCTCAAACTTGG-3' (the sequence identical to the region from positions 923 to 943 is linked with the complementary sequence to the region from positions 981 to 1000);
SEQ ID NO:36:
   5'-GCGCAGTATGAGGAGATGGCCAGAGGGTCTCAAACTTGGTCT-3' (the sequence identical to the region from positions 923 to 943 is linked with the complementary sequence to the region from positions 978 to 997);
SEQ ID NO:37:
   5'-GGTCAAGGCGCAGTATGAGGAGGGGTCTCAAACTTGGTCTGG-3' (the sequence identical to the region from positions 916 to 937 is linked with the complementary sequence to the region from positions 976 to 995);
SEQ ID NO:38:
   5'-CACCTACCGCAAGCTGCTGGTCACAGAGATATTCACGGCT-3' (the sequence identical to the region from positions 1297 to 1316 is linked with the complementary sequence to the region from positions 1354 to 1376);
SEQ ID NO:39:
   5'-GCCAAATGCAGCCGGGCTTGGAGGGTCTCAAACTTGGTCTGGTA-3' (the sequence identical to the region from positions 941 to 958 is linked with the complementary sequence to the region from positions 974 to 999);
SEQ ID NO:40:
   5'-GGACGGCATCATCGCTGAGGGTCTCAAACTTGGTCTGGTA-3' (the sequence identical to the region from positions 898 to 917 is linked with the complementary sequence to the region from positions 978 to 993);
SEQ ID NO:41:
   5'-TTCAGAGATGAACCGGGCCATCCACGCTGGTTCTTGATGTTGT-3' (the sequence identical to the region from positions 1051 to 1072 is linked with the complementary sequence to the region from positions 1095 to 1115);
SEQ ID NO:42:
   5'-TTCAGAGATGAACCGGGCCATCGGCACGCTGGTTCTTGA-3' (the sequence identical to the region from positions 1051 to 1072 is linked with the complementary sequence to the region from positions 1101 to 1117);
SEQ ID NO:43:
   5'-TTCAGAGATGAACCGGGCCATCGGCACGCTGGTTCTTGAT-3' (the sequence identical to the region from positions 1051 to 1072 is linked with the complementary sequence to the region from positions 1100 to 1117);
SEQ ID NO:44:
   5'-CACCTACCGCAAGCTGCTGGATCATCACAGAGATATTCACGGCTC-3' (the sequence identical to the region from positions 1297 to 1317 is linked with the complementary sequence to the region from positions 1353 to 1376); and
SEQ ID NO:45:
   5'-CCGCAAGCTGCTGGAGGGAATTCATCACAGAGATATTCACGGCTC-3' (the sequence identical to the region from positions 1303 to 1320 is linked with the complementary sequence to the region from positions 1353 to 1379).

### (R3 primer)

SEQ ID NO:46: 5'-CAGCTCCACCTTGCTCATG-3' (the complementary sequence to the region from positions 742 to 760);
SEQ ID NO:47: 5'-AGCTCCACCTTGCTCATGT-3' (the complementary sequence to the region from positions 741 to 759);
SEQ ID NO:48: 5'-AGGTCGTCCCCATGCTTC-3' (the complementary sequence to the region from positions 1012 to 1029);
SEQ ID NO:49: 5'-CGTCCCCATGCTTCCCA-3' (the complementary sequence to the region from positions 1009 to 1025);
SEQ ID NO:50: 5'-TCCCCATGCTTCCCAGC-3' (the complementary sequence to the region from positions 1007 to 1023);
SEQ ID NO:51: 5'-CACCGCCACTGCTACTG-3' (the complementary sequence to the region from positions 1390 to 1406);
SEQ ID NO:52: 5'-CGTCCCCATGCTTCCCA-3' (the complementary sequence to the region from positions 1009 to 1025);
SEQ ID NO:53: 5'-GGTCGTCCCCATGCTTCC-3' (the complementary sequence to the region from positions 1011 to 1028);
SEQ ID NO:54: 5'-GGTCGTCCCCATGCTTC-3' (the complementary sequence to the region from positions 1012 to 1028);
SEQ ID NO:55: 5'-GGCGGCCTCCAACTTG-3' (the complementary sequence to the region from positions 1117 to 1132);
SEQ ID NO:56: 5'-TGGCGGCCTCCAACTT-3' (the complementary sequence to the region from positions 1118 to 1133);
SEQ ID NO:57: 5'-AATGGCGGCCTCCAACTT-3' (the complementary sequence to the region from positions 1118 to 1135);
SEQ ID NO:58: 5'-CCACTGCTACTGCCACCA-3' (the complementary sequence to the region from positions 1384 to 1401);
SEQ ID NO:59: 5'-ATGGCGGCCTCCAACTT-3' (the complementary sequence to the region from positions 1118 to 1134); and
SEQ ID NO:60: 5'-CACTGCTACTGCCACCAG-3' (the complementary sequence to the region from positions 1383 to 1400).

### (Loop primer F)

SEQ ID NO:61: 5'-GGGCCTGGAGGGTCTCAAA-3' (the complementary sequence to the region from positions 986 to 1004);
SEQ ID NO:62: 5'-TGGGCCTGGAGGGTCTCAA-3' (the complementary sequence to the region from positions 987 to 1005);
SEQ ID NO:63: 5'-GGGCCTGGAGGGTCTCAAACT-3' (the complementary sequence to the region from positions 984 to 1004);
SEQ ID NO:64: 5'-GGGCCTGGAGGGTCTCAAAC-3' (the complementary sequence to the region from positions 985 to 1004);
SEQ ID NO:65: 5'-GGCCTGGAGGGTCTCAAACT-3' (the complementary sequence to the region from positions 984 to 1003);
SEQ ID NO:66: 5'-GGCCTGGAGGGTCTCAAACTT-3' (the complementary sequence to the region from positions 983 to 1003);
SEQ ID NO:67: 5'-CTGGGCCTGGAGGGTCTCAA-3' (the complementary sequence to the region from positions 987 to 1006);
SEQ ID NO:68: 5'-TGGGCCTGGAGGGTCTCAAA-3' (the complementary sequence to the region from positions 986 to 1005);
SEQ ID NO:69: 5'-GCCTGGAGGGTCTCAAACT-3' (the complementary sequence to the region from positions 984 to 1002);
SEQ ID NO:70: 5'-TGGGCCTGGAGGGTCTCAA-3' (the complementary sequence to the region from positions 987 to 1005);
SEQ ID NO:71: 5'-GCCTGGAGGGTCTCAAACTT-3' (the complementary sequence to the region from positions 983 to 1002);
SEQ ID NO:72: 5'-TCACGCTCATGAGTTCCTGGT-3' (the complementary sequence to the region from positions 1251 to 1271);
SEQ ID NO:73: 5'-GCTTCACGCTCATGAGTTCCTG-3' (the complementary sequence to the region from positions 1253 to 1274);
SEQ ID NO:74: 5'-GCTTCACGCTCATGAGTTCCTGGT-3' (the complementary sequence to the region from positions 1251 to 1274);
SEQ ID NO:75: 5'-CTTCACGCTCATGAGTTCCTGG-3' (the complementary sequence to the region from positions 1252 to 1273);
SEQ ID NO:76: 5'-GCCAGCTTCACGCTCATGAG-3' (the complementary sequence to the region from positions 1259 to 1278);
SEQ ID NO:77: 5'-GCCAGCTTCACGCTCATGAGTTC-3' (the complementary sequence to the region from positions 1256 to 1278);
SEQ ID NO:78: 5'-CTTCACGCTCATGAGTTCCTGGT-3' (the complementary sequence to the region from positions 1251 to 1273);
SEQ ID NO:79: 5'-GGCCAGCTTCACGCTCATGA-3' (the complementary sequence to the region from positions 1260 to 1279);
SEQ ID NO:80: 5'-CCAGCTTCACGCTCATGAGTTC-3' (the complementary sequence to the region from positions 1256 to 1277);
SEQ ID NO:81: 5'-GCCAGCTTCACGCTCATGAGTT-3' (the complementary sequence to the region from positions 1257 to 1278);
SEQ ID NO:82: 5'-GCCAGCTTCACGCTCATGAGT-3' (the complementary sequence to the region from positions 1258 to 1278);
SEQ ID NO:83: 5'-GCTTCACGCTCATGAGTTCCTGG-3' (the complementary sequence to the region from positions 1252 to 1274); and
SEQ ID NO:84: 5'-GGCCAGCTTCACGCTCATGAG-3' (the complementary sequence to the region from positions 1259 to 1279).

### (Loop primer R)

SEQ ID NO:85: 5'-AGGCTGCAGGCTGAGATCG-3' (the sequence identical to the region from positions 1076 to 1094);
SEQ ID NO:86: 5'-GAGGCTGCAGGCTGAGATCG-3' (the sequence identical to the region from positions 1076 to 1093);
SEQ ID NO:87: 5'-AGAGGCTGCAGGCTGAGATC-3' (the sequence identical to the region from positions 1074 to 1093);
SEQ ID NO:88: 5'-GAGGCTGCAGGCTGAGATCG-3' (the sequence identical to the region from positions 1075 to 1094);
SEQ ID NO:89: 5'-CAGAGGCTGCAGGCTGA-3' (the sequence identical to the region from positions 1073 to 1089);
SEQ ID NO:90: 5'-CAGAGGCTGCAGGCTGAGA-3' (the sequence identical to the region from positions 1073 to 1091);
SEQ ID NO:91: 5'-CAGAGGCTGCAGGCTGAGATC-3' (the sequence identical to the region from positions 1073 to 1093);
SEQ ID NO:92: 5'-GCTGCAGGCTGAGATCGACAAC-3' (the sequence identical to the region from positions 1078 to 1099);
SEQ ID NO:93: 5'-GCAGGCTGAGATCGACAACA-3' (the sequence identical to the region from positions 1081 to 1100);
SEQ ID NO:94: 5'-GCTGCAGGCTGAGATCGACA-3' (the sequence identical to the region from positions 1078 to 1097);
SEQ ID NO:95: 5'-GCTGCAGGCTGAGATCGACAA-3' (the sequence identical to the region from positions 1078 to 1098);
SEQ ID NO:96: 5'-GGCTGCAGGCTGAGATCGAC-3' (the sequence identical to the region from positions 1077 to 1096);
SEQ ID NO:97: 5'-CCGGTTGGCTGGAGATGGAGTG-3' (the sequence identical to the region from positions 1330 to 1351);
SEQ ID NO:98: 5'-AGCCGGTTGGCTGGAGAT-3' (the sequence identical to the region from positions 1328 to 1345);
SEQ ID NO:99: 5'-CGGTTGGCTGGAGATGGAGTGG-3' (the sequence identical to the region from positions 1331 to 1352);
SEQ ID NO:100: 5'-CCGGTTGGCTGGAGATGGAG-3' (the sequence identical to the region from positions 1330 to 1349);
SEQ ID NO:101: 5'-CGGTTGGCTGGAGATGGAGT-3' (the sequence identical to the region from positions 1331 to 1350);
SEQ ID NO:102: 5'-CGGTTGGCTGGAGATGGAGTG-3' (the sequence identical to the region from positions 1331 to 1351);
SEQ ID NO:103: 5'-GGTTGGCTGGAGATGGAGTGG-3' (the sequence identical to the region from positions 1332 to 1352); and
SEQ ID NO:104: 5'-CCGGTTGGCTGGAGATGGA-3' (the sequence identical to the region from positions 1330 to 1348).

The following primers can also be used, in addition to the above primers.

### (F3 primer)

SEQ ID NO:105: 5'-TTGCCGAGGCTGAGGA-3' (the sequence identical to the region from positions 1134 to 1149).

### (FIP)

SEQ ID NO:106:
   5'-CGTCCCCATGCTTCCCAGCCTGAAGCCTGGTACCAGACC-3' (the complementary sequence to the region from positions 1006 to 1025 is linked with the sequence identical to the region from positions 964 to 982);
SEQ ID NO:107:
   5'-CGTCCCCATGCTTCCCAGCTGAAGCCTGGTACCAGACC-3' (the complementary sequence to the region from positions 1007 to 1025 is linked with the sequence identical to the region from positions 964 to 982); and
SEQ ID NO:108:
   5'-CTGCCGTGCCATATCCTGCTTGGCTCAAGGATGCTCGTGC-3' (the complementary sequence to the region from positions 1219 to 1240 is linked with the sequence identical to the region from positions 1165 to 1182).

### (RIP)

SEQ ID NO:109:
   5'-CGCAGTATGAGGAGATGGCCAACTGGAGGGTCTCAAACTTGG-3' (the sequence identical to the region from positions 924 to 945 is linked with the complementary sequence to the region from positions 981 to 1000);
SEQ ID NO:110:
   5'-ATGAACCGGGCCATCCAGAGGCTTGGCACGCTGGTTCTTG-3' (the sequence identical to the region from positions 1058 to 1078 is linked with the complementary sequence to the region from positions 1102 to 1120);
SEQ ID NO:111:
   5'-TTCAGAGATGAACCGGGCCATCACTTGGCACGCTGGTTCTTG-3' (the sequence identical to the region from positions 1051 to 1072 is linked with the complementary sequence to the region from positions 1102 to 1121);
SEQ ID NO:112:
   5'-TCAGAGATGAACCGGGCCATCCACTTGGCACGCTGGTTCTTG-3' (the sequence identical to the region from positions 1052 to 1073 is linked with the complementary sequence to the region from positions 1102 to 1121);
SEQ ID NO:113:
   5'-TTCAGAGATGAACCGGGCCATCCACGCTGGTTCTTGATGTTGT-3' (the sequence identical to the region from positions 1051 to 1072 is linked with the complementary sequence to the region from positions 1095 to 1115);
SEQ ID NO:114:
   5'-TCAGAGATGAACCGGGCCATCCCACGCTGGTTCTTGATGTTGT-3' (the sequence identical to the region from positions 1052 to 1073 is linked with the complementary sequence to the region from positions 1095 to 1115); and
SEQ ID NO:115:
   5'-GTGAAGCTGGCCCTGGACACCAACCGGCTCTCCTC-3' (the sequence identical to the region from positions 1268 to 1286 is linked with the complementary sequence to the region from positions 1322 to 1337).

### (R3 primer)

SEQ ID NO:116: 5'-GAGGTCGTCCCCATGCTTC-3' (the complementary sequence to the region from positions 1012 to 1030);
SEQ ID NO:117: 5'-CTCAGCCTCGGCAATGG-3' (the complementary sequence to the region from positions 1131 to 1147);
SEQ ID NO:118: 5'-CCTCAGCCTCGGCAATG-3' (the complementary sequence to the region from positions 1132 to 1148);
SEQ ID NO:119: 5'-TGGCGGCCTCCAACTTG-3' (the complementary sequence to the region from positions 1117 to 1133); and
SEQ ID NO:120: 5'-GCTCCCACTCCATCTCCA-3' (the complementary sequence to the region from positions 1339 to 1356).

### Among the above RIP primers, the primers having SEQ ID NOs:3 and 4 are capable of hybridizing to a region containing the exon junction 3.

The primers having SEQ ID NOs:35 to 37, 39, 40 and 109 are capable of hybridizing to a region containing the exon junction 5.

The primers having SEQ ID NOs:41 to 43 and 110 to 114 are capable of hybridizing to a region containing the exon junction 6.

The primers having SEQ ID NO:115 is capable of hybridizing to a region containing the exon junction 7.

The primers having SEQ ID NOs:38, 44 and 45 are capable of hybridizing to a region containing the exon junction 8.

The above primers will be used as a primer set in which the primers are appropriately combined according to the targeted amplification region(s). Specific examples of the primer set including four primers, F3 primer, FIP, RIP and R3 primer, are listed in Table 1. In Table 1, the numbers indicated in each column for the respective primers correspond to the Sequence ID numbers.

**[Table 1]**

| **Primer set** | **F3 primer** | FIP | RIP | **R3 primer** |
|---|---|---|---|---|
| 1 | 2 | 14 | 33 | 46 |
| 2 | 3 | 15 | 34 | 46 |
| 3 | 4 | 14 | 34 | 47 |
| 4 | 5 | 16 | 35 | 48 |
| 5 | 5 | 20 | 109 | 116 |
| 6 | 6 | 17 | 36 | 49 |
| 7 | 7 | 18 | 37 | 50 |
| 8 | 12 | 26 | 42 | 56 |
| 9 | 12 | 106 | 43 | 59 |
| 10 | 12 | 107 | 43 | 57 |
| 11 | 12 | 25 | 110 | 117 |
| 12 | 12 | 25 | 111 | 118 |
| 13 | 12 | 106 | 112 | 117 |
| 14 | 12 | 25 | 113 | 55 |
| 15 | 12 | 106 | 114 | 119 |
| 16 | 105 | 108 | 115 | 120 |
| 17 | 8 | 19 | 38 | 51 |
| 18 | 9 | 21 | 39 | 52 |
| 19 | 9 | 22 | 39 | 53 |
| 20 | 10 | 23 | 39 | 52 |
| 21 | 11 | 24 | 40 | 54 |

Specific examples of the primer set including, in addition to F3 primer, FIP, RIP and R3 primer, and loop primers F and R are listed in Table 2. In Table 2, the numbers indicated in each column for the respective primers correspond to the Sequence ID numbers.

**[Table 2]**

| **Primer set** | **F3 primer** | FIP | RIP | **R3 primer** | **Loop primer F** | Loop primer R | | **Primer set** | **F3 primer** | FIP | RIP | **R3 primer** | **Loop primer F** | Loop primer R |
|---|---|---|---|---|---|---|---|---|---|---|---|---|---|---|
| 22 | 12 | 26 | 42 | 56 | 70 | 96 | | 42 | 12 | 25 | 41 | 55 | 64 | 88 |
| 23 | 12 | 26 | 42 | 56 | 66 | 85 | | 43 | 12 | 25 | 41 | 55 | 61 | 85 |
| 24 | 12 | 26 | 42 | 56 | 66 | 93 | | 44 | 12 | 25 | 41 | 55 | 62 | 86 |
| 25 | 12 | 26 | 42 | 56 | 69 | 88 | | 45 | 13 | 30 | 44 | 58 | 72 | 98 |
| 26 | 12 | 26 | 42 | 56 | 65 | 89 | | 46 | 13 | 30 | 44 | 58 | 72 | 100 |
| 27 | 12 | 28 | 42 | 56 | 65 | 91 | | 47 | 13 | 30 | 44 | 58 | 72 | 103 |
| 28 | 12 | 27 | 43 | 59 | 66 | 91 | | 48 | 13 | 29 | 44 | 58 | 71 | 97 |
| 29 | 12 | 27 | 43 | 59 | 66 | 92 | | 49 | 13 | 29 | 44 | 58 | 71 | 99 |
| 30 | 12 | 27 | 43 | 59 | 65 | 90 | | 50 | 13 | 29 | 44 | 58 | 73 | 97 |
| 31 | 12 | 27 | 43 | 59 | 65 | 88 | | 51 | 13 | 29 | 44 | 58 | 74 | 97 |
| 32 | 12 | 27 | 43 | 57 | 63 | 92 | | 52 | 13 | 29 | 44 | 58 | 74 | 99 |
| 33 | 12 | 27 | 43 | 57 | 64 | 94 | | 53 | 13 | 31 | 45 | 60 | 81 | 103 |
| 34 | 12 | 27 | 43 | 57 | 68 | 88 | | 54 | 13 | 31 | 45 | 60 | 75 | 100 |
| 35 | 12 | 27 | 43 | 57 | 62 | 95 | | 55 | 13 | 31 | 45 | 60 | 83 | 104 |
| 36 | 12 | 27 | 43 | 57 | 67 | 88 | | 56 | 13 | 31 | 45 | 60 | 78 | 103 |
| 37 | 12 | 25 | 41 | 55 | 66 | 90 | | 57 | 13 | 31 | 45 | 60 | 79 | 101 |
| 38 | 12 | 25 | 41 | 55 | 66 | 88 | | 58 | 13 | 31 | 45 | 60 | 76 | 101 |
| 39 | 12 | 25 | 41 | 55 | 65 | 91 | | 59 | 13 | 31 | 45 | 60 | 80 | 102 |
| 40 | 12 | 25 | 41 | 55 | 65 | 85 | | 60 | 13 | 32 | 45 | 60 | 77 | 102 |
| 41 | 12 | 25 | 41 | 55 | 63 | 87 | | 61 | 13 | 32 | 45 | 60 | 82 | 102 |

In RT-LAMP method, CK7 mRNA in a sample can be detected by mixing and reacting the sample, the primer set of the present embodiment, RNA-dependent DNA polymerase (hereinafter referred to as "reverse transcriptase"), DNA-dependent DNA polymerase having strand displacement activity (hereinafter referred to as "strand displacement type DNA polymerase") and dNTPs (comprising dATP, dGTP, dTTP and dCTP).

The reverse transcriptase and strand displacement type DNA polymerase to be used can be the ones well-known in the art. Instead of the reverse transcriptase and strand displacement type DNA polymerase, one enzyme may also be used which can synthesize DNA from RNA as a template and synthesize DNA from DNA as a template by strand displacement.

In the above enzyme reaction, it is preferable to use a buffer which can provide a suitable reaction condition.
The primer set of the present embodiment can be provided as a reagent in the freeze-dried form or in the solution form. Different primers may be contained in the same container or in the respective different containers.
The reagents used in RT-LAMP method may be contained in the respective different containers or the reverse transcriptase and strand displacement type DNA polymerase may be contained in the same container.
At least two of dNTPs, buffer and primers may be contained in the same container. When providing these reagents to users, a reagent kit comprising some or all of these reagents may be provided.
The sample subjected to the detection of CK7 mRNA may include tissue obtained from a biological body (e.g. lymph node, lymph fluid, blood), feces and the like.

The detection by RT-LAMP is carried out through an amplification step and a detection step. First, a sample obtained from a biological body containing CK7 mRNA is mixed with the primer set, reverse transcriptase, dNTPs and strand displacement type DNA polymerase. The mixture is heated to a certain temperature (e.g. 65°C) to carry out RT and LAMP reactions, so that CK7 cDNA is amplified.

Mechanisms of RT and LAMP reactions are described below.
In a reaction mixture, the second sequence in RIP first hybridizes to the region R2c in CK7 mRNA. The reverse transcriptase synthesizes CK7 cDNA from 3'-end of RIP using CK7 mRNA as a template. A double strand nucleic acid of CK7 mRNA and CK7 cDNA which is extended from RIP (RIP extended strand) is synthesized.
R3 primer then hybridizes to the region R3c in CK7 mRNA. The strand replacement type DNA polymerase synthesizes CK7 cDNA from 3'-end of R3 primer, using CK7 mRNA as a template, by separating (displacing) the RIP extended strand from CK7 mRNA.
The RIP extended strand has the sequence complementary to CK7 mRNA, so that it contains the region F2c. Due to the above reaction, the RIP extended strand is now a single strand. The fourth sequence of FIP hybridizes to this single strand.
The strand displacement type DNA polymerase synthesizes DNA from 3'-end of FIP using the RIP extended strand as a template. The thus synthesized DNA has the third sequence (a sequence hybridizing to the region F1) in its 5'-end, which hybridizes to the region F1 in the synthesized DNA to form a stem-loop structure. The synthesized DNA also has a complementary sequence to the first sequence (a sequence hybridizing to the region R1c) in its 3'-end, which hybridizes to the region R1 c in the synthesized DNA to form a stem-loop structure. Accordingly, the synthesized DNA forms a dumbbell-like structure having stem-loop structures in both 5'- and 3'-ends.
The strand displacement type DNA polymerase extends this dumbbell-like structure DNA from 3'-end by unwinding the stem-loop structure in 5'-end and using its own sequence as a template. The thus extended strand has sequences complementary to each other in its 5'-end, 3'-end and between these ends, so that the extended strand forms three stem-loop structures. Further, the strand displacement type DNA polymerase extends the extended strand from 3'-end by unwinding the stem-loop structures and using its own sequence as a template. By repeating these reactions under an isothermal condition, DNA molecules which respectively comprise multiple specific sequences are synthesized (details for the reactions can be found in USP 6,410,278 and USP 6,974,670).

Most of the nucleic acids thus synthesized are dsDNAs (double strand DNAs). Therefore, the amplification products can be detected by staining the products with a fluorescent intercalater such as ethidium bromide, SYBR GREEN I, Pico Green and irradiating ultraviolet rays. The fluorescent staining may be carried out by adding a fluorescent dye in the reaction solution after the amplification. Alternatively, the fluorescent dye may be added to the reaction solution before carrying out the nucleic acid amplification reaction in the presence of the fluorescent dye. The presence or absence of the fluorescence detection allows the assessment of the presence or absence of CK7 mRNA in a sample. By measuring the fluorescent intensity of the reaction solution, the amplified products can be quantified after the reaction. Based on the quantification result, CK7 mRNA in the sample can also be quantified. In particular, when nucleic acid amplification is carried out in the presence of the fluorescent dye, the increase in the fluorescent intensity of the reaction solution and the time period until when the fluorescent intensity reaches to a certain level can be measured in real time, allowing the calculation of the amount of CK7 mRNA in the sample (real-time RT-PCR, real-time RT-LAMP and the like).

In LAMP reaction, a by-product magnesium pyrophosphate is produced which is insoluble in water. The further the nucleic acid amplification proceeds, the more the amount of magnesium pyrophosphate is and the more cloudy the reaction solution is. Thus, by visually confirming the cloudiness of the reaction solution, or measuring the turbidity of the reaction solution via absorbance or scattered light intensity, or filtering the reaction solution through a colored filter and confirming a residue on the filter, the target nucleic acids can be detected (see WO 01/83817). When the turbidity is measured by measuring the absorbance or scattered light intensity of the reaction solution, the amplification of DNA and turbidity can be followed in a closed system by monitoring the change in turbidity in real time, as similar to the case of using the fluorescent dye. The presence or absence of cloudiness of the reaction solution allows the assessment of the presence or absence of CK7 mRNA in a sample. By measuring the turbidity of the reaction solution, the amplified product can be quantified. Based on the quantification result, CK7 mRNA in the sample can also be quantified. When the turbidity is measured in real time, the time period until when the turbidity reaches to a certain level (hereinafter referred to as "rise time") can be measured to convert this time period into the amount of CK7 mRNA in the sample.

### EXAMPLES

### Example 1

RT-LAMP method was carried out with the primer sets 1 to 21 shown in Table 1, in order to demonstrate that CK7 mRNA can be detected.

### (1) Preparation of sample

RNA (5 x 10¹⁰ copies/µL) was serially diluted in an RNase-free Yeast RNA solution (containing 50ng/µL Yeast RNA (Ambion)) to prepare 5x10³ copies/µL RNA (hereinafter referred to as "RNA sample A").

### (2) Preparation of reaction solutions

F3primer, FIP, RIP and R3 primer were added to a buffer as follows to prepare a primer mix:
32)µM FIP;
32µM RIP;
2µMF3 primer;
2µMR3 primer and
10 mM Tris-HCl (pH8.0).

RT-LAMP reaction buffer was prepared by mixing the following reagents:
53 mM Tris-HCl (pH8.8);
1.8 x Thermopol buffer (New England Biolabs);
1.43 mM dNTPs (Invitrogen);
5.36 mM MgSO₄ (Nacalai Tesque);
8.93 mM DTT (Wako Pure Chemical Industries); and
2% Tergitol (Sigma).

Enzyme solution was prepared by mixing the following reagents:
10U/µL AMV reverse transcriptase (Promega);
8U/µL BstDNA polymerase (New England Biolabs); and
RNase inhibitor (Promega).

Reaction solution (25µl) was prepared by mixing the following reagents:

| | | |
|---|---|---|
| RT-LAMP reaction buffer | | 14.0µL |
| Enzyme solution | | 3.0µL |
| Primer mix | | 2.5µL |
| 10 mM Tris-HCl (pH8.0) | 3.5µL | |
| RNA sample A | | 2.0µL. |

As a negative control (NC), a reaction solution was also prepared in which 2.0µl of Yeast RNA solution (containing 50 ng/µL Yeast RNA (Ambion)) was added instead of 2.0µL of RNA sample A.

### (3) Detection and results

Tubes containing the reaction solutions with either of 21 primer sets were placed in a real-time turbidimeter LA-200 (TERAMECS) preliminarily heated to 65°C and incubated at the same temperature. The time was measured from the start of the reaction until the turbidity of the reaction solution reached to 0.1. The measurement for negative control was carried out once and the measurement for RNA sample A was carried out for three times (n = 3). The results of negative control and the average values for RNA sample A are described in Table 3.

**[Table 3]**

| **Primer set** | NC **(min.)** | **Results** **(min.)** |
|---|---|---|
| 1 | - | 31.1 |
| 2 | - | 35.2 |
| 3 | - | 27.9 |
| 4 | - | 38.4 |
| 5 | - | 53.7 |
| 6 | - | 48.8 |
| 7 | - | 50.0 |
| 8 | - | 29.9 |
| 9 | - | 35.6 |
| 10 | - | 27.8 |
| 11 | - | 39.4 |
| 12 | - | 28.4 |
| 13 | - | 41.8 |
| 14 | - | 26.3 |
| 15 | - | 41.2 |
| 16 | - | 48.7 |
| 17 | - | 55.3 |
| 18 | - | 30.5 |
| 19 | - | 30.6 |
| 20 | - | 34.3 |
| 21 | - | 36.9 |

In Table 3, "-" means that the turbidity did not reach to 0.1 within 60 minutes. As shown in Table 3, no turbidity was detected within 60 minutes in negative controls (i.e. no non-specific amplification was occurred). However, the nucleic acid amplification was confirmed within 60 minutes for three measurements of RNA sample A (the average values are shown in Table 3). These results show that CK7 mRNA can be detected within 60 minutes by using any of primer sets 1 to 21.

### Example 2

RT-LAMP method was carried out with the primer sets 22 to 61 shown in Table 2, in order to demonstrate that CK7 mRNA can be detected.

### (1) Preparation of sample

RNA (5 x 10¹⁰ copies/µL) was serially diluted with an RNase-free Yeast RNA solution (containing 50ng/µL Yeast RNA (Ambion)) to prepare 2.5 x 10³ copies/µL RNA (hereinafter referred to as "RNA sample B").

### (2) Preparation of reaction solutions

F3primer, FIP, RIP, R3 primer and loop primers F and R were added to a buffer as follows to prepare a primer mix:
16µM FIP;
16µM RIP;
1µM F3 primer;
1µM R3 primer;
12µM Loop primer F;
12µM Loop primer R; and
10 mM Tris-HCl (pH8.0).

Reaction solution (25µl) was prepared by mixing the following reagents:

| | | |
|---|---|---|
| RT-LAMP reaction buffer of Example 1 | 14.0µL | |
| Enzyme solution of Example 1 | 3.0µL | |
| Primer mix | | 5µL |
| 10 mM Tris-HCl (pH8.0) | 1.0µL | |
| RNA sample B | | 2.0µL. |

### (3) Detection and results

Tubes containing the reaction solutions with either of 40 primer sets were placed in a real-time turbidimeter LA-200 (TERAMECS) preliminarily heated to 65°C and incubated at the same temperature. The time was measured from the start of the reaction until the turbidity of the reaction solution reached 0.1. The measurement for negative control was carried out once and the measurement for RNA sample B was carried out for three times (n = 3). The results of negative control and the average values for RNA sample B are described in Table 4.

**[Table 4]**

| **Primer set** | NC **(min.)** | **Results** (min.) | | **Primer set** | NC **(min.)** | **Results** **(min.)** |
|---|---|---|---|---|---|---|
| 22 | - | 14.6 | | 42 | - | 12.8 |
| 23 | - | 13.6 | | 43 | - | 12.4 |
| 24 | - | 14.0 | | 44 | - | 12.6 |
| 25 | - | 14.2 | | 45 | - | 16.1 |
| 26 | - | 13.3 | | 46 | - | 17.2 |
| 27 | - | 13.8 | | 47 | - | 26.0 |
| 28 | - | 16.0 | | 48 | - | 15.0 |
| 29 | - | 16.5 | | 49 | - | 16.9 |
| 30 | - | 23.3 | | 50 | - | 16.8 |
| 31 | - | 16.6 | | 51 | - | 17.2 |
| 32 | - | 13.9 | | 52 | - | 18.0 |
| 33 | - | 14.1 | | 53 | - | 27.6 |
| 34 | - | 13.9 | | 54 | - | 22.5 |
| 35 | - | 14.3 | | 55 | - | 33.6 |
| 36 | - | 13.7 | | 56 | - | 25.6 |
| 37 | - | 13.3 | | 57 | - | 26.2 |
| 38 | - | 13.2 | | 58 | - | 22.9 |
| 39 | - | 14.0 | | 59 | - | 27.1 |
| 40 | - | 13.1 | | 60 | - | 24.1 |
| 41 | - | 12.7 | | 61 | - | 30.2 |

In Table 4, "-" means that the turbidity did not reach to 0.1 within 60 minutes. As shown in Table 4, no turbidity was detected within 60 minutes in negative controls (i.e. no non-specific amplification was occurred). However, the nucleic acid amplification was confirmed within 40 minutes for three measurements of RNA sample B (the average values are shown in Table 4). These results show that CK7 mRNA can be detected within 40 minutes by using any of primer sets 22 to 61.

The present application relates to Japanese Patent Application No. 2008-088188 filed on March 28, 2008, whose claims, specification, drawing, tables, Sequence Listing and abstract are entirely incorporated herein by reference.

## Claims

1. A reagent comprising primers for the detection of cytokeratin-7 mRNA,
which comprises a first primer, a second primer, a third primer and a fourth primer, wherein:
1) the first primer comprises a first sequence and a second sequence in its 5' and 3' regions, respectively,
the first sequence (a) being 10 to 30 nucleotides long; and (b) being capable of hybridizing to a complementary strand to a first region in the sequence SEQ ID NO:1,
the second sequence (c) being 10 to 30 nucleotides long; and (d) being capable of hybridizing to a second region located at 3' to the first region in the sequence SEQ ID NO:1,
the second region comprising:
i. nucleotides at positions 724 and 725 in SEQ ID NO:1;
ii. nucleotides at positions 985 and 986 in SEQ ID NO:1;
iii. nucleotides at positions 1111 and 1112 in SEQ ID NO:1;
iv. nucleotides at positions 1332 and 1333 in SEQ ID NO:1; or
v. nucleotides at positions 1367 and 1368 in SEQ ID NO:1;
2) the second primer comprises a third sequence and a fourth sequence in its 5' and 3' regions, respectively,
the third sequence (e) being 10 to 30 nucleotides long; and (f) being capable of hybridizing to a third region located at 5' to the first region in the sequence SEQ ID NO:1,
the fourth sequence (g) being 10 to 30 nucleotides long; and (h) being capable of hybridizing to a fourth region located at 5' to the third region in the sequence SEQ ID NO:1,
3) the third primer is capable of hybridizing to a fifth region located at 5' to the fourth region in the sequence SEQ ID NO:1, and
4) the fourth primer is capable of hybridizing to a sixth region located at 3' to the second region in the sequence SEQ ID NO:1.

2. A primer for the detection of cytokeratin-7 mRNA comprising a first sequence and a second sequence in its 5' and 3' regions, respectively,
wherein the first sequence (a) is 10 to 30 nucleotides long; and (b) is capable of hybridizing to a complementary strand to a first region in the sequence SEQ ID NO:1,
the second sequence (c) is 10 to 30 nucleotides long; and (d) is capable of hybridizing to a second region located at 3' to the first region in the sequence SEQ ID NO:1, and
the second region comprises:
i. nucleotides at positions 724 and 725 in SEQ ID NO:1;
ii. nucleotides at positions 985 and 986 in SEQ ID NO:1;
iii. nucleotides at positions 1111 and 1112 in SEQ ID NO:1;
iv. nucleotides at positions 1332 and 1333 in SEQ ID NO:1; or
v. nucleotides at positions 1367 and 1368 in SEQ ID NO:1.

3. The primer according to claim 2, which is selected from the group consisting of:
(a) a polynucleotide of any of SEQ ID NOs:33 to 45 and 109 to 115; and
(b) a polynucleotide having a nucleotide sequence of the polynucleotide of (a) from or to which one of more nucleotides have been substituted, deleted, inserted or added and having primer function in nucleic acid amplification reaction.

4. The primer according to claim 2 or 3, which hybridizes to a region in SEQ ID NO:1 selected from the group consisting of:
the region from positions 721 to 739;
the region from positions 974 to 999;
the region from positions 976 to 995;
the region from positions 978 to 993;
the region from positions 978 to 997;
the region from positions 981 to 1000;
the region from positions 1095 to 1115;
the region from positions 1100 to 1117;
the region from positions 1101 to 1117;
the region from positions 1353 to 1376;
the region from positions 1353 to 1379; and
the region from positions 1354 to 1376.

5. The primer according to claim 2, wherein three nucleotides at 3'-end of the second sequence are completely complementary to a sequence of the second region.

6. A primer set for the detection of cytokeratin-7 mRNA, comprising a first primer, a second primer, a third primer and a fourth primer,
wherein:
1) the first primer is the primer according to claim 2;
2) the second primer comprises a third sequence and a fourth sequence in its 5' and 3' regions, respectively,
the third sequence (i) being 10 to 30 nucleotides long; and (ii) being capable of hybridizing to a third region located at 5' to the first region in the sequence SEQ ID NO:1,
the fourth sequence (iii) being 10 to 30 nucleotides long; and (iv) being capable of hybridizing to a fourth region located at 5' to the third region in the sequence SEQ ID NO:1,
3) the third primer is capable of hybridizing to a fifth region located at 5' to the fourth region in the sequence SEQ ID NO:1, and
4) the fourth primer is capable of hybridizing to a sixth region located at 3' to the second region in the sequence SEQ ID NO:1.

7. The primer set according to claim 6, wherein the second primer is selected from the group consisting of:
(a) a polynucleotide of any of SEQ ID NOs:14 to 32 and 106 to 108; and
(b) a polynucleotide having a nucleotide sequence of the polynucleotide of (a) from or to which one of more nucleotides have been substituted, deleted, inserted or added and having primer function in nucleic acid amplification reaction, and
the third primer is selected from the group consisting of:
(c) a polynucleotide of any of SEQ ID NOs:2 to 13 and 105; and
(d) a polynucleotide having a nucleotide sequence of the polynucleotide of (c) from or to which one of more nucleotides have been substituted, deleted, inserted or added and having primer function in nucleic acid amplification reaction.

8. The primer set according to claim 6, wherein the second primer hybridizes to a complementary region to a region in SEQ ID NO:1 selected from the group consisting of:
the region from positions 574 to 590;
the region from positions 813 to 834;
the region from positions 813 to 882;
the region from positions 816 to 834;
the region from positions 837 to 856;
the region from positions 840 to 857;
the region from positions 851 to 869;
the region from positions 851 to 871;
the region from positions 861 to 882;
the region from positions 863 to 883;
the region from positions 963 to 982;
the region from positions 964 to 982;
the region from positions 1165 to 1182;
the region from positions 1235 to 1252; and
the region from positions 1236 to 1252.

9. The primer set according to claim 6, wherein the third primer hybridizes to a region in SEQ ID NO:1 selected from the group consisting of:
the region from positions 538 to 556;
the region from positions 542 to 561;
the region from positions 546 to 564;
the region from positions 775 to 794;
the region from positions 796 to 815;
the region from positions 816 to 834;
the region from positions 827 to 843;
the region from positions 828 to 845;
the region from positions 840 to 859;
the region from positions 932 to 950;
the region from positions 1134 to 1149; and
the region from positions 1213 to 1228.

10. The primer set according to claim 6, wherein the fourth primer is selected from the group consisting of:
(a) a polynucleotide of any of SEQ ID NOs:46 to 60 and 116 to 120; and
(b) a polynucleotide having a nucleotide sequence of the polynucleotide of (a) from or to which one of more nucleotides have been substituted, deleted, inserted or added and having primer function in nucleic acid amplification reaction.

11. The primer set according to claim 6, wherein the fourth primer hybridizes to a region in SEQ ID NO:1 selected from the group consisting of:
the region from positions 741 to 759;
the region from positions 742 to 760;
the region from positions 1007 to 1023;
the region from positions 1009 to 1025;
the region from positions 1011 to 1028;
the region from positions 1012 to 1028;
the region from positions 1012 to 1029;
the region from positions 1012 to 1030;
the region from positions 1117 to 1132;
the region from positions 1117 to 1133;
the region from positions 1118 to 1133;
the region from positions 1118 to 1134;
the region from positions 1118 to 1135;
the region from positions 1131 to 1147;
the region from positions 1132 to 1148;
the region from positions 1356 to 1339;
the region from positions 1383 to 1400;
the region from positions 1384 to 1401; and
the region from positions 1390 to 1406.

12. The primer set according to claim 6, which further comprises a fifth primer hybridizing to a seventh region located between the third region and the fourth region in the sequence SEQ ID NO:1.

13. The primer set according to claim 12, wherein the fifth primer is selected from the group consisting of:
(a) a polynucleotide of any of SEQ ID NOs:61 to 84; and
(b) a polynucleotide having a nucleotide sequence of the polynucleotide of (a) from or to which one of more nucleotides have been substituted, deleted, inserted or added and having primer function in nucleic acid amplification reaction.

14. The primer set according to claim 12, wherein the fifth primer hybridizes to a region in SEQ ID NO:1 selected from the group consisting of:
the region from positions 983 to 1002;
the region from positions 983 to 1003;
the region from positions 984 to 1002;
the region from positions 984 to 1003;
the region from positions 984 to 1004;
the region from positions 985 to 1004;
the region from positions 986 to 1004;
the region from positions 986 to 1005;
the region from positions 987 to 1005;
the region from positions 987 to 1006;
the region from positions 1251 to 1271;
the region from positions 1251 to 1273;
the region from positions 1251 to 1274;
the region from positions 1252 to 1273;
the region from positions 1252 to 1274;
the region from positions 1253 to 1274;
the region from positions 1256 to 1277;
the region from positions 1256 to 1278;
the region from positions 1257 to 1278;
the region from positions 1258 to 1278;
the region from positions 1259 to 1278;
the region from positions 1259 to 1279; and
the region from positions 1260 to 1279.

15. The primer set according to claim 6, which further comprises a sixth primer hybridizing to a complementary region to a eighth region located between the first region and the second region in the sequence SEQ ID NO:1.

16. The primer set according to claim 15, wherein the sixth primer is selected from the group consisting of:
(a) a polynucleotide of any of SEQ ID NOs:85 to 104; and
(b) a polynucleotide having a nucleotide sequence of the polynucleotide of (a) from or to which one of more nucleotides have been substituted, deleted, inserted or added and having primer function in nucleic acid amplification reaction.

17. The primer set according to claim 15, wherein the sixth primer hybridizes to a complementary region to a region in SEQ ID NO:1 selected from the group consisting of:
the region from positions 1073 to 1089;
the region from positions 1073 to 1091;
the region from positions 1073 to 1093;
the region from positions 1074 to 1093;
the region from positions 1075 to 1094;
the region from positions 1076 to 1093;
the region from positions 1076 to 1094;
the region from positions 1077 to 1096;
the region from positions 1078 to 1097;
the region from positions 1078 to 1098;
the region from positions 1078 to 1099;
the region from positions 1081 to 1100;
the region from positions 1328 to 1345;
the region from positions 1330 to 1348;
the region from positions 1330 to 1349;
the region from positions 1330 to 1351;
the region from positions 1331 to 1350;
the region from positions 1331 to 1351;
the region from positions 1331 to 1352; and
the region from positions 1332 to 1352.

18. A primer for the detection of cytokeratin-7 mRNA comprising the sequence SEQ ID NO:25.

19. A kit for the detection of cytokeratin-7 mRNA comprising the primer set according to claim 6, a RNA-dependent DNA polymerase, a DNA-dependent DNA polymerase and dNTPs.

20. A method of detecting cytokeratin-7 mRNA comprising the steps of:
1) reacting a sample obtained from a biological body, the primer set according to claim 6 and a RNA-dependent DNA polymerase to synthesize cDNA from cytokeratin-7 mRNA in the sample;
2) reacting the primer set, a DNA-dependent DNA polymerase and cDNA synthesized in the step 1) to amplify cDNA; and
3) detecting the amplified cDNA to detect cytokeratin-7 mRNA in the sample.

## Amended claims

### Amended claims under Art. 19.1 PCT

1. A reagent comprising primers for the detection of cytokeratin-7 mRNA,
which comprises a first primer, a second primer, a third primer and a fourth primer, wherein:
1) the first primer comprises a first sequence and a second sequence in its 5' and 3' regions, respectively,
the first sequence (a) being 10 to 30 nucleotides long; and (b) being capable of hybridizing to a complementary strand to a first region in the sequence SEQ ID NO:1,
the second sequence (c) being 10 to 30 nucleotides long; and (d) being capable of hybridizing to a second region located at 3' to the first region in the sequence SEQ ID NO:1,
the second region comprising:
i. nucleotides at positions 724 and 725 in SEQ ID NO:1;
ii. nucleotides at positions 985 and 986 in SEQ ID NO:1;
iii. nucleotides at positions 1111 and 1112 in SEQ ID NO:1;
iv. nucleotides at positions 1332 and 1333 in SEQ ID NO:1; or
v. nucleotides at positions 1367 and 1368 in SEQ ID NO:1;
2) the second primer comprises a third sequence and a fourth sequence in its 5' and 3' regions, respectively,
the third sequence (e) being 10 to 30 nucleotides long; and (f) being capable of hybridizing to a third region located at 5' to the first region in the sequence SEQ ID NO:1,
the fourth sequence (g) being 10 to 30 nucleotides long; and (h) being capable of hybridizing to a complementary strand to a fourth region located at 5' to the third region in the sequence SEQ ID NO:1,
3) the third primer is capable of hybridizing to a complementary strand to a fifth region located at 5' to the fourth region in the sequence SEQ ID NO:1, and
4) the fourth primer is capable of hybridizing to a sixth region located at 3' to the second region in the sequence SEQ ID NO:1.

2. A primer for the detection of cytokeratin-7 mRNA comprising a first sequence and a second sequence in its 5' and 3' regions, respectively,
wherein the first sequence (a) is 10 to 30 nucleotides long; and (b) is capable of hybridizing to a complementary strand to a first region in the sequence SEQ ID NO:1,
the second sequence (c) is 10 to 30 nucleotides long; and (d) is capable of hybridizing to a second region located at 3' to the first region in the sequence SEQ ID NO:1, and
the second region comprises:
i. nucleotides at positions 724 and 725 in SEQ ID NO:1;
ii. nucleotides at positions 985 and 986 in SEQ ID NO:1;
iii. nucleotides at positions 1111 and 1112 in SEQ ID NO:1;
iv. nucleotides at positions 1332 and 1333 in SEQ ID NO:1; or
v. nucleotides at positions 1367 and 1368 in SEQ ID NO:1.

3. The primer according to claim 2, which is selected from the group consisting of:
(a) a polynucleotide of any of SEQ ID NOs:33 to 45 and 109 to 115; and
(b) a polynucleotide having a nucleotide sequence of the polynucleotide of (a) from or to which one of more nucleotides have been substituted, deleted, inserted or added and having primer function in nucleic acid amplification reaction.

4. The primer according to claim 2 or 3, which hybridizes to a region in SEQ ID NO:1 selected from the group consisting of:
the region from positions 721 to 739;
the region from positions 974 to 999;
the region from positions 976 to 995;
the region from positions 978 to 993;
the region from positions 978 to 997;
the region from positions 981 to 1000;
the region from positions 1095 to 1115;
the region from positions 1100 to 1117;
the region from positions 1101 to 1117;
the region from positions 1353 to 1376;
the region from positions 1353 to 1379; and
the region from positions 1354 to 1376.

5. The primer according to claim 2, wherein three nucleotides at 3'-end of the second sequence are completely complementary to a sequence of the second region.

6. A primer set for the detection of cytokeratin-7 mRNA, comprising a first primer, a second primer, a third primer and a fourth primer,
wherein:
1) the first primer is the primer according to claim 2;
2) the second primer comprises a third sequence and a fourth sequence in its 5' and 3' regions, respectively,
the third sequence (i) being 10 to 30 nucleotides long; and (ii) being capable of hybridizing to a third region located at 5' to the first region in the sequence SEQ ID NO:1,
the fourth sequence (iii) being 10 to 30 nucleotides long; and (iv) being capable of hybridizing to a complementary strand to a fourth region located at 5' to the third region in the sequence SEQ ID NO:1,
3) the third primer is capable of hybridizing to a complementary strand to a fifth region located at 5' to the fourth region in the sequence SEQ ID NO:1, and
4) the fourth primer is capable of hybridizing to a sixth region located at 3' to the second region in the sequence SEQ ID NO:1.

7. The primer set according to claim 6, wherein the second primer is selected from the group consisting of:
(a) a polynucleotide of any of SEQ ID NOs:14 to 32 and 106 to 108; and
(b) a polynucleotide having a nucleotide sequence of the polynucleotide of (a) from or to which one of more nucleotides have been substituted, deleted, inserted or added and having primer function in nucleic acid amplification reaction, and the third primer is selected from the group consisting of:
(c) a polynucleotide of any of SEQ ID NOs:2 to 13 and 105; and
(d) a polynucleotide having a nucleotide sequence of the polynucleotide of (c) from or to which one of more nucleotides have been substituted, deleted, inserted or added and having primer function in nucleic acid amplification reaction.

8. The primer set according to claim 6, wherein the second primer hybridizes to a complementary region to a region in SEQ ID NO:1 selected from the group consisting of:
the region from positions 574 to 590;
the region from positions 813 to 834;
the region from positions 813 to 882;
the region from positions 816 to 834;
the region from positions 837 to 856;
the region from positions 840 to 857;
the region from positions 851 to 869;
the region from positions 851 to 871;
the region from positions 861 to 882;
the region from positions 863 to 883;
the region from positions 963 to 982;
the region from positions 964 to 982;
the region from positions 1165 to 1182;
the region from positions 1235 to 1252; and
the region from positions 1236 to 1252.

9. The primer set according to claim 6, wherein the third primer hybridizes to a complementary strand to a region in SEQ ID NO:1 selected from the group consisting of:
the region from positions 538 to 556;
the region from positions 542 to 561;
the region from positions 546 to 564;
the region from positions 775 to 794;
the region from positions 796 to 815;
the region from positions 816 to 834;
the region from positions 827 to 843;
the region from positions 828 to 845;
the region from positions 840 to 859;
the region from positions 932 to 950;
the region from positions 1134 to 1149; and
the region from positions 1213 to 1228.

10. The primer set according to claim 6, wherein the fourth primer is selected from the group consisting of:
(a) a polynucleotide of any of SEQ ID NOs:46 to 60 and 116 to 120; and
(b) a polynucleotide having a nucleotide sequence of the polynucleotide of (a) from or to which one of more nucleotides have been substituted, deleted, inserted or added and having primer function in nucleic acid amplification reaction.

11. The primer set according to claim 6, wherein the fourth primer hybridizes to a region in SEQ ID NO:1 selected from the group consisting of:
the region from positions 741 to 759;
the region from positions 742 to 760;
the region from positions 1007 to 1023;
the region from positions 1009 to 1025;
the region from positions 1011 to 1028;
the region from positions 1012 to 1028;
the region from positions 1012 to 1029;
the region from positions 1012 to 1030;
the region from positions 1117 to 1132;
the region from positions 1117 to 1133;
the region from positions 1118 to 1133;
the region from positions 1118 to 1134;
the region from positions 1118 to 1135;
the region from positions 1131 to 1147;
the region from positions 1132 to 1148;
the region from positions 1356 to 1339;
the region from positions 1383 to 1400;
the region from positions 1384 to 1401; and
the region from positions 1390 to 1406.

12. The primer set according to claim 6, which further comprises a fifth primer hybridizing to a seventh region located between the third region and the fourth region in the sequence SEQ ID NO:1.

13. The primer set according to claim 12, wherein the fifth primer is selected from the group consisting of:
(a) a polynucleotide of any of SEQ ID NOs:61 to 84; and
(b) a polynucleotide having a nucleotide sequence of the polynucleotide of (a) from or to which one of more nucleotides have been substituted, deleted, inserted or added and having primer function in nucleic acid amplification reaction.

14. The primer set according to claim 12, wherein the fifth primer hybridizes to a region in SEQ ID NO:1 selected from the group consisting of:
the region from positions 983 to 1002;
the region from positions 983 to 1003;
the region from positions 984 to 1002;
the region from positions 984 to 1003;
the region from positions 984 to 1004;
the region from positions 985 to 1004;
the region from positions 986 to 1004;
the region from positions 986 to 1005;
the region from positions 987 to 1005;
the region from positions 987 to 1006;
the region from positions 1251 to 1271;
the region from positions 1251 to 1273;
the region from positions 1251 to 1274;
the region from positions 1252 to 1273;
the region from positions 1252 to 1274;
the region from positions 1253 to 1274;
the region from positions 1256 to 1277;
the region from positions 1256 to 1278;
the region from positions 1257 to 1278;
the region from positions 1258 to 1278;
the region from positions 1259 to 1278;
the region from positions 1259 to 1279; and
the region from positions 1260 to 1279.

15. The primer set according to claim 6, which further comprises a sixth primer hybridizing to a complementary region to a eighth region located between the first region and the second region in the sequence SEQ ID NO:1.

16. The primer set according to claim 15, wherein the sixth primer is selected from the group consisting of:
(a) a polynucleotide of any of SEQ ID NOs:85 to 104; and
(b) a polynucleotide having a nucleotide sequence of the polynucleotide of (a) from or to which one of more nucleotides have been substituted, deleted, inserted or added and having primer function in nucleic acid amplification reaction.

17. The primer set according to claim 15, wherein the sixth primer hybridizes to a complementary region to a region in SEQ ID NO:1 selected from the group consisting of:
the region from positions 1073 to 1089;
the region from positions 1073 to 1091;
the region from positions 1073 to 1093;
the region from positions 1074 to 1093;
the region from positions 1075 to 1094;
the region from positions 1076 to 1093;
the region from positions 1076 to 1094;
the region from positions 1077 to 1096;
the region from positions 1078 to 1097;
the region from positions 1078 to 1098;
the region from positions 1078 to 1099;
the region from positions 1081 to 1100;
the region from positions 1328 to 1345;
the region from positions 1330 to 1348;
the region from positions 1330 to 1349;
the region from positions 1330 to 1351;
the region from positions 1331 to 1350;
the region from positions 1331 to 1351;
the region from positions 1331 to 1352; and
the region from positions 1332 to 1352.

18. The primer set according to claim 6, wherein the second primer comprises the sequence SEQ ID NO:25.

19. A kit for the detection of cytokeratin-7 mRNA comprising the primer set according to claim 6, a RNA-dependent DNA polymerase, a DNA-dependent DNA polymerase and dNTPs.

20. A method of detecting cytokeratin-7 mRNA comprising the steps of:
1) reacting a sample obtained from a biological body, the primer set according to claim 6 and a RNA-dependent DNA polymerase to synthesize cDNA from cytokeratin-7 mRNA in the sample;
2) reacting the primer set, a DNA-dependent DNA polymerase and cDNA synthesized in the step 1) to amplify cDNA; and
3) detecting the amplified cDNA to detect cytokeratin-7 mRNA in the sample.
